(19) Europäisches Patentamt / European Patent Office / Office européen des brevets

(11) EP 3 751 004 A1

(12) **EUROPEAN PATENT APPLICATION**
published in accordance with Art. 153(4) EPC

(43) Date of publication:
**16.12.2020 Bulletin 2020/51**

(21) Application number: **19751298.1**

(22) Date of filing: **04.02.2019**

(51) Int Cl.:
***C12P 19/34*** *(2006.01)* ***C12N 15/11*** *(2006.01)*
***C12N 9/00*** *(2006.01)* ***C12N 15/52*** *(2006.01)*

(86) International application number:
**PCT/JP2019/003827**

(87) International publication number:
**WO 2019/156020 (15.08.2019 Gazette 2019/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.02.2018 JP 2018021799**

(71) Applicant: **Sumitomo Chemical Company, Limited**
**Tokyo 104-8260 (JP)**

(72) Inventor: **SAKATA Akihiro**
**Osaka-shi, Osaka 555-0021 (JP)**

(74) Representative: **J A Kemp LLP**
**14 South Square**
**Gray's Inn**
**London WC1R 5JJ (GB)**

(54) **MANUFACTURING METHOD FOR NUCLEIC ACID MOLECULE**

(57) A manufacturing method for a single-stranded RNA includes a step of causing an RNA ligase to act on a first single-stranded RNA having a phosphate group at the 5' end and a second single-stranded RNA having a hydroxyl group at the 3' end to link the first single-stranded RNA and the second single-stranded RNA. The first single-stranded RNA is a single-stranded RNA consisting of an X1 region and a Z region in order from the 5' end; the second single-stranded RNA is a single strand consisting of an X2 region, a Y2 region, a Ly linker region, and a Y1 region in order from the 5' end ; the X1 region and the X2 region have nucleotide sequences which are complementary to each other, have a length of at least two nucleotides, and have the same number of nucleotides; and the Y1 region and the Y2 region have nucleotide sequences which are complementary to each other, have a length of at least two nucleotides, and have the same number of nucleotides.

FIG. 1

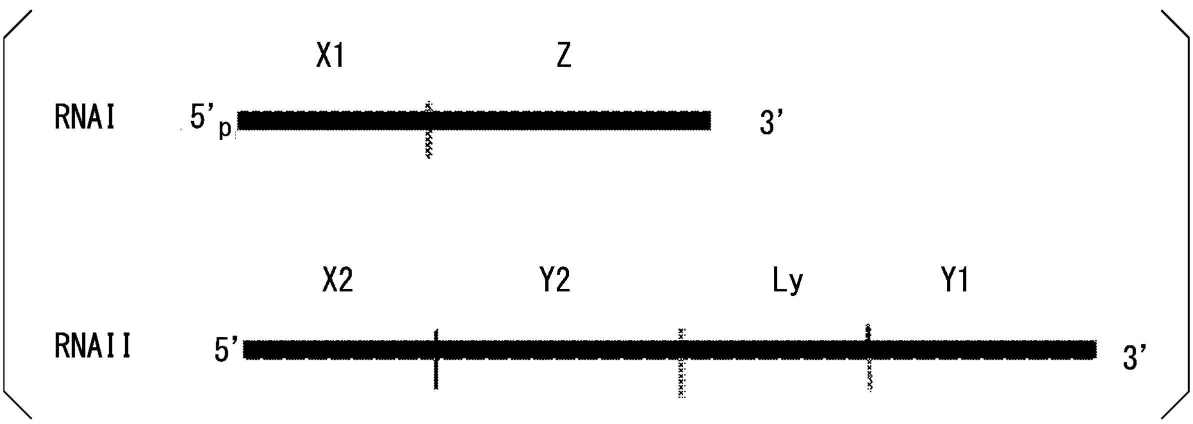


EP 3 751 004 A1

SEQUENCE LISTING

<110> Sumitomo Chemical Co., Ltd.

<120> Method for producing nucleic acid molecule

<130> PC26899

<150> JP2018-21799
<151> 2018-02-09

<160> 23

<170> PatentIn version 3.5

<210> 1
<211> 13
<212> RNA
<213> Artificial Sequence

<220>
<223> synthesized RNA

<400> 1
guguacucug cuu 13

<210> 2
<211> 23
<212> RNA
<213> Artificial Sequence

<220>
<223> synthesized RNA

<400> 2
gcagaguaca cacagcauau acc 23

<210> 3
<211> 12
<212> RNA
<213> Artificial Sequence

<220>
<223> synthesized RNA

<400> 3
gguauaugcu gu 12

<210> 4
<211> 14
<212> RNA
<213> Artificial Sequence

<220>
<223> synthesized RNA

<400> 4
guguacucug cuuc 14

<210> 5
<211> 24
<212> RNA
<213> Artificial Sequence

<220>
<223> synthesized RNA

<400> 5
agcagaguac acacagcaua uacc 24

<210> 6
<211> 12
<212> RNA
<213> Artificial Sequence

<220>
<223> synthesized RNA

<400> 6
gguauaugcu gu 12

<210> 7
<211> 18
<212> RNA
<213> Artificial Sequence

<220>
<223> synthesized RNA

<400> 7
cuguguguac ucugcuuc 18

<210> 8
<211> 24
<212> RNA
<213> Artificial Sequence

<220>
<223> synthesized RNA

<400> 8
agcagaguac acacagcaua uacc 24

<210> 9
<211> 20
<212> RNA
<213> Artificial Sequence

<220>
<223> synthesized RNA

<400> 9
ugcugugugu acucugcuuc 20

<210> 10
<211> 24
<212> RNA

<213> Artificial Sequence

<220>
<223> synthesized RNA

<400> 10
agcagaguac acacagcaua uacc 24

<210> 11
<211> 22
<212> RNA
<213> Artificial Sequence

<220>
<223> synthesized RNA

<400> 11
uaugcugugu guacucugcu uc 22

<210> 12
<211> 24
<212> RNA
<213> Artificial Sequence

<220>
<223> synthesized RNA

<400> 12
agcagaguac acacagcaua uacc 24

<210> 13
<211> 24
<212> RNA
<213> Artificial Sequence

<220>
<223> synthesized RNA

<400> 13
uauaugcugu guguacucug cuuc 24

<210> 14
<211> 24
<212> RNA
<213> Artificial Sequence

<220>
<223> synthesized RNA

<400> 14
agcagaguac acacagcaua uacc 24

<210> 15
<211> 25
<212> RNA
<213> Artificial Sequence

<220>

<223> synthesized RNA

<400> 15
guauaugcug uguguacucu gcuuc 25

<210> 16
<211> 24
<212> RNA
<213> Artificial Sequence

<220>
<223> synthesized RNA

<400> 16
agcagaguac acacagcaua uacc 24

<210> 17
<211> 23
<212> RNA
<213> Artificial Sequence

<220>
<223> synthesized RNA

<400> 17
gcagaguaca cacagcauau acc 23

<210> 18
<211> 25
<212> RNA
<213> Artificial Sequence

<220>
<223> synthesized RNA

<400> 18
gguauaugcu guguguacuc ugcuu 25

<210> 19
<211> 24
<212> RNA
<213> Artificial Sequence

<220>
<223> synthesized RNA

<400> 19
agcagaguac acacagcaua uacc 24

<210> 20
<211> 26
<212> RNA
<213> Artificial Sequence

<220>
<223> synthesized RNA

<400> 20

gguauaugcu guguguacuc ugcuuc 26

<210> 21
<211> 334
<212> PRT
<213> bacteriophage T4

<400> 21

```
Met Phe Lys Lys Tyr Ser Ser Leu Glu Asn His Tyr Asn Ser Lys Phe
1               5                   10                  15

Ile Glu Lys Leu Tyr Ser Leu Gly Leu Thr Gly Gly Glu Trp Val Ala
            20                  25                  30

Arg Glu Lys Ile His Gly Thr Asn Phe Ser Leu Ile Ile Glu Arg Asp
        35                  40                  45

Lys Val Thr Cys Ala Lys Arg Thr Gly Pro Ile Leu Pro Ala Glu Asp
    50                  55                  60

Phe Phe Gly Tyr Glu Ile Ile Leu Lys Asn Tyr Ala Asp Ser Ile Lys
65                  70                  75                  80

Ala Val Gln Asp Ile Met Glu Thr Ser Ala Val Val Ser Tyr Gln Val
                85                  90                  95

Phe Gly Glu Phe Ala Gly Pro Gly Ile Gln Lys Asn Val Asp Tyr Cys
            100                 105                 110

Asp Lys Asp Phe Tyr Val Phe Asp Ile Ile Val Thr Thr Glu Ser Gly
        115                 120                 125

Asp Val Thr Tyr Val Asp Asp Tyr Met Met Glu Ser Phe Cys Asn Thr
    130                 135                 140

Phe Lys Phe Lys Met Ala Pro Leu Leu Gly Arg Gly Lys Phe Glu Glu
145                 150                 155                 160

Leu Ile Lys Leu Pro Asn Asp Leu Asp Ser Val Val Gln Asp Tyr Asn
                165                 170                 175

Phe Thr Val Asp His Ala Gly Leu Val Asp Ala Asn Lys Cys Val Trp
            180                 185                 190

Asn Ala Glu Ala Lys Gly Glu Val Phe Thr Ala Glu Gly Tyr Val Leu
        195                 200                 205

Lys Pro Cys Tyr Pro Ser Trp Leu Arg Asn Gly Asn Arg Val Ala Ile
```

210 215 220

Lys Cys Lys Asn Ser Lys Phe Ser Glu Lys Lys Lys Ser Asp Lys Pro
225 230 235 240

Ile Lys Ala Lys Val Glu Leu Ser Glu Ala Asp Asn Lys Leu Val Gly
245 250 255

Ile Leu Ala Cys Tyr Val Thr Leu Asn Arg Val Asn Asn Val Ile Ser
260 265 270

Lys Ile Gly Glu Ile Gly Pro Lys Asp Phe Gly Lys Val Met Gly Leu
275 280 285

Thr Val Gln Asp Ile Leu Glu Glu Thr Ser Arg Glu Gly Ile Thr Leu
290 295 300

Thr Gln Ala Asp Asn Pro Ser Leu Ile Lys Lys Glu Leu Val Lys Met
305 310 315 320

Val Gln Asp Val Leu Arg Pro Ala Trp Ile Glu Leu Val Ser
325 330

<210> 22
<211> 335
<212> PRT
<213> Vibrio phage KVP40

<400> 22

Met Ser Phe Val Lys Tyr Thr Ser Leu Glu Asn Ser Tyr Arg Gln Ala
1 5 10 15

Phe Val Asp Lys Cys Asp Met Leu Gly Val Arg Glu Trp Val Ala Leu
20 25 30

Glu Lys Ile His Gly Ala Asn Phe Ser Phe Ile Val Glu Phe Lys Pro
35 40 45

Asn Glu Ala Gln Asp Gly Ala Glu Phe Thr Val Thr Pro Ala Lys Arg
50 55 60

Thr Ser Thr Ile Gly Ala Asn Val Met Gly Asp Tyr Asp Phe Tyr Gly
65 70 75 80

Cys Thr Ser Val Val Glu Ala His Thr Ala Lys Met Glu Ala Ile Ser
85 90 95

Asn Trp Leu Trp Ala Arg Gly Ile Ile Asn Val Gly Glu Thr Ile Ile

```
            100                 105                 110

Val Tyr Gly Glu Leu Ala Gly Lys Gly Val Gln Lys Glu Val Asn Tyr
        115                 120                 125

Gly Asp Lys Asp Phe Trp Ala Tyr Asp Ile Leu Leu Pro Glu Thr Gly
    130                 135                 140

Lys Phe Leu Asp Trp Asp Val Val Leu Glu Ala Cys Glu Phe Ala Lys
145                 150                 155                 160

Val Lys Thr Thr His Glu Ile Ala Arg Gly Thr Leu Asp Glu Leu Leu
                165                 170                 175

Arg Ile Asp Pro Leu Phe Arg Ser Phe His Thr Pro Ala Asp Val Asp
            180                 185                 190

Gly Asp Asn Val Ala Glu Gly Phe Val Val Lys Gln Leu Arg Asn Glu
        195                 200                 205

Lys Arg Leu His Asn Gly Ser Arg Ala Ile Leu Lys Val Lys Asn Asp
    210                 215                 220

Lys Phe Lys Glu Lys Lys Asn Lys Ala Gly Lys Thr Pro Arg Ala Ala
225                 230                 235                 240

Val Val Leu Thr Glu Glu Gln Glu Lys Leu His Ala Ala Phe Ser Cys
                245                 250                 255

Tyr Leu Thr Glu Asn Arg Leu Arg Asn Val Leu Ser Lys Leu Glu Thr
            260                 265                 270

Val Thr Gln Lys Gln Phe Gly Met Ile Ser Gly Leu Phe Ile Lys Asp
        275                 280                 285

Ala Lys Asp Glu Phe Glu Arg Asp Glu Leu Asn Glu Thr Ala Ile Ala
    290                 295                 300

Arg Asp Asp Trp Asp Val Ile Lys Arg Ser Leu Thr Asn Ile Ala Asn
305                 310                 315                 320

Glu Ile Leu Arg Lys Asn Trp Leu Asp Ile Leu Asp Gly Asn Phe
                325                 330                 335
```

<210> 23
<211> 342
<212> PRT
<213> Deinococcus radiorurans

<400> 23

Met Ala Glu Arg Gln Val Ile Lys Glu Arg Ala Gln Leu Phe Pro His
1               5                   10                  15

Pro Asn Ala Glu Arg Leu Glu Leu Cys Lys Val Gly Thr Phe Gln Leu
            20                  25                  30

Val Val Arg Lys Gly Glu Tyr Arg Asp Gly Asp Pro Ile Val Ile Ala
        35                  40                  45

Pro Glu Lys Ala Val Leu Pro Pro Gln Leu Ala Gly Leu Tyr Thr Asn
    50                  55                  60

Ala Asp Thr Gly Ala Ser Tyr Leu His Gly Ala Glu Lys Asn Arg Val
65                  70                  75                  80

Gly Ser Val Arg Leu Arg Gly Glu Val Ser Gln Gly Val Ile Leu Pro
                85                  90                  95

Leu Asp Gly Leu Glu Asp Ala Pro Phe Gly Glu Asp Leu Ala Glu Arg
            100                 105                 110

Leu Gly Ile Thr Phe Trp Glu Pro Pro Val Pro Val Ser Met Ala Gly
        115                 120                 125

Glu Val Glu Pro Arg Pro Pro Ala Gln His Tyr Lys His His Asp Val
    130                 135                 140

Glu Gln Phe Gly Ile Tyr Val Ser Glu Phe Ala Ser Gly Glu Glu Val
145                 150                 155                 160

Met Val Thr Glu Lys Leu His Gly Thr Gln Gly Val Tyr Phe Arg Thr
                165                 170                 175

Ala Glu Gly Arg Trp Leu Val Thr Ser Lys Gly Leu Ser Arg Gly Gly
            180                 185                 190

Leu Thr Leu Arg Glu Ala Ala Ser Asn Val Tyr Trp Gln Ala Ala Arg
        195                 200                 205

Asn Ser Asn Leu Phe Ala Glu Ala Asp Ala Ala Phe Ser Gly Gly Glu
    210                 215                 220

Val Gln Ile Phe Gly Glu Val Val Pro Val Gln Lys Gly Phe Ser Tyr
225                 230                 235                 240

```
Gly Gln His Lys Pro Thr Val Phe Val Phe Lys Val Val Tyr Asp Gly
                245                 250                 255

Leu Arg Leu Pro Arg Arg Asp Trp Pro Gln Trp Val Leu Asp His Ala
            260                 265                 270

Val Pro Val Leu Tyr Glu Gly Pro Phe Asp Glu Ala Thr Val Arg Lys
        275                 280                 285

Leu Arg Gly Gly Leu Glu Thr Val Ser Gly Lys Gly Leu His Ile Arg
    290                 295                 300

Glu Gly Val Val Val Ala Pro Lys Val Pro Arg Phe Ala Ala Asp Gly
305                 310                 315                 320

Ser Asp Leu Ser Val Lys Leu Ile Ser Asp Ala Tyr Ala Lys Lys Glu
                325                 330                 335

Thr Gly Glu Glu Tyr Ser
            340
```

## Description

[Technical Field]

The present invention relates to a manufacturing method for a nucleic acid molecule.

Priority is claimed on Japanese Patent Application No. 2018-21799, filed February 9, 2018, the content of which is incorporated herein by reference.

[Background Art]

Patent Documents 1 and 2 disclose a single-stranded RNA used for an RNA interference method, and as a manufacturing method for such compound, a method for manufacturing the compound by a multistage chemical reaction using a nucleic acid synthesizer has been known.

[Citation List]

[Patent Document]

[Patent Document 1]
PCT International Publication No. WO2012/017919
[Patent Document 2]
PCT International Publication No. WO2013/103146

[Summary of Invention]

[Technical Problem]

An object of the present invention is to provide a simple manufacturing method for a single-stranded RNA.

[Solution to Problem]

The present invention includes the following aspects.

A manufacturing method for a single-stranded RNA according to an aspect of the present invention is a manufacturing method for a single-stranded RNA including a step of causing an RNA ligase which is classified as EC 6.5.1.3 defined by the International Union of Biochemistry and Molecular Biology as an enzyme number and has a double strand nick repair activity to act on a first single-stranded RNA having a phosphate group at the 5' end and a second single-stranded RNA having a hydroxyl group at the 3' end to link the first single-stranded RNA and the second single-stranded RNA, in which the manufacturing method comprises the following features a) to g):

a) the first single-stranded RNA is a single-stranded RNA consisting of an X1 region and a Z region in order from the 5' end;
b) the second single-stranded RNA is a single strand consisting of an X2 region, a Y2 region, a Ly linker region, and a Y1 region in order from the 5' end;
c) the X1 region and the X2 region comprise nucleotide sequences which are complementary to each other and have the same number of nucleotides of two or more;
d) the Y1 region and the Y2 region comprise nucleotide sequences which are complementary to each other and have the same number of nucleotides of two or more;
e) the Z region is a region which comprises a nucleotide sequence having any number of nucleotides;
f) the Ly linker region is a linker region having an atomic group derived from an amino acid; and
g) a single-stranded RNA generated by linking the first single-stranded RNA and the second single-stranded RNA is a linked single-stranded RNA consisting of the X2 region, the Y2 region, the Ly linker region, the Y1 region, the X1 region, and the Z region from the 5' end.

In one aspect of the present invention, there is provided a manufacturing method according to the method as described above, wherein the Z region is a region consisting of a Z1 region, an Lz linker region, and a Z2 region in order from the 5' end, the Lz linker region is a linker region having an atomic group derived from an amino acid, the Zlregion

and the Z2 region comprise nucleotide sequences complementary to each other, and the single-stranded RNA generated by linking the first single-stranded RNA and the second single-stranded RNA is a linked single-stranded RNA consisting of the X2 region, the Y2 region, the Ly linker region, the Y1 region, the X1 region, the Z1 region, the Lz linker region, and the Z2 region in order from the 5' end.

In one aspect of the present invention, there is provided a manufacturing method for a single-stranded RNA according to the manufacturing method as described above, wherein the Ly linker region is a divalent group represented by Formula (I).

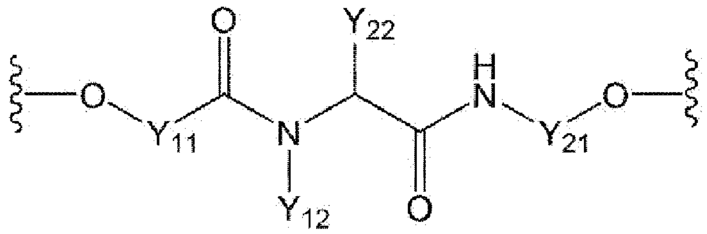

(I)

In the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms; the terminal oxygen atom bonded to $Y_{11}$ is bonded to a phosphorus atom of a phosphate ester of a terminal nucleotide in any one region of the Y1 region and the Y2 region; and the terminal oxygen atom bonded to $Y_{21}$ is bonded to a phosphorus atom of a phosphate ester of a terminal nucleotide in the other region of the Y1 region and the Y2 region, which is not bonded to $Y_{11}$.

In one aspect of the present invention, there is provided a manufacturing method for a single-stranded RNA according to the manufacturing method as described above is a manufacturing method, wherein the Ly linker region is a divalent group represented by Formula (I), and the Lz linker region is a divalent group represented by Formula (I').

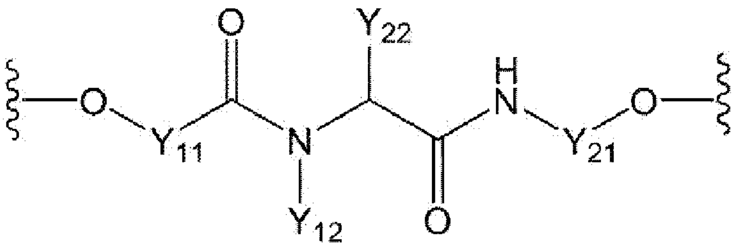

(I)

In the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms; the terminal oxygen atom bonded to $Y_{11}$ is bonded to a phosphorus atom of a phosphate ester of a terminal nucleotide in any one region of the Y1 region and the Y2 region; and the terminal oxygen atom bonded to $Y_{21}$ is bonded to a phosphorus atom of a phosphate ester of a terminal nucleotide in the other region of the Y2 region and the Y1 region, which is not bonded to $Y_{11}$.

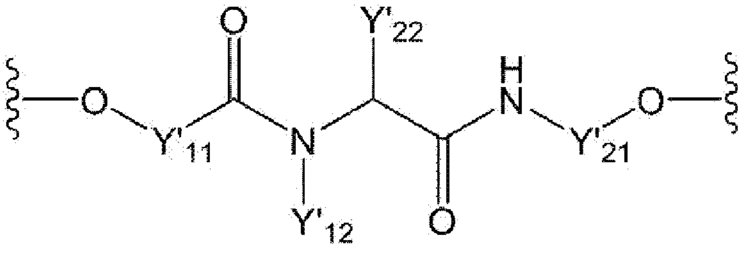

(I')

In the formula, $Y'_{11}$ and $Y'_{21}$ each independently represent an alkylene group having 1 to 20 carbon atoms, and $Y'_{12}$ and $Y'_{22}$ each independently represent a hydrogen atom or an alkyl group which may be substituted with an amino group, or $Y'_{12}$ and $Y'_{22}$ are bonded to each other at their terminals to represent an alkylene group having 3 or 4 carbon atoms; the terminal oxygen atom bonded to $Y'_{11}$ is bonded to a phosphorus atom of a phosphate ester of a

terminal nucleotide in any one region of the Z1 region and the Z2 region; and the terminal oxygen atom bonded to $Y'_{21}$ is bonded to a phosphorus atom of a phosphate ester of a terminal nucleotide in the other region of the Z2 region and the Z1 region, which is not bonded to $Y'_{11}$.)

In one aspect of the present invention, there is provided a manufacturing method for a single-stranded RNA according to the manufacturing method as described above, wherein the Ly linker region and the Lz linker region are each independently a divalent group having a structure represented by Formula (II-A) or (II-B).

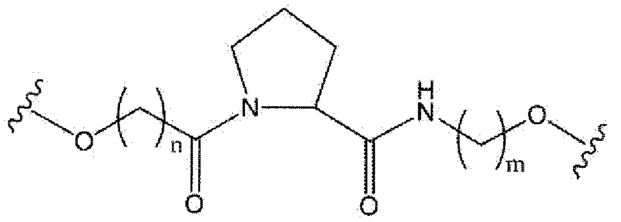

(II-A)

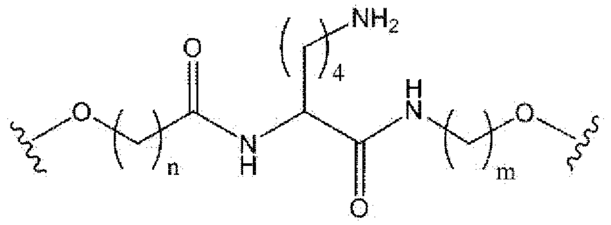

(II-B)

In the formulae, n and m each independently represent any integer of 1 to 20.

In one aspect of the present invention, there is provided a manufacturing method for a single-stranded RNA according to the manufacturing method as described above, wherein at least one of a W1 region consisting of the X1 region, the Y1 region, and the Z region and a W2 region consisting of the X2 region and the Y2 region has a nucleotide sequence that suppresses expression of a gene targeted by an RNA interference method.

In one aspect of the present invention, there is provided a manufacturing method for a single-stranded RNA according to the manufacturing method as described above, wherein the RNA ligase is a T4 bacteriophage-derived T4 RNA ligase 2, a KVP40-derived ligase 2, a Trypanosoma brucei RNA ligase, a Deinococcus radiodurans RNA ligase, or a Leishmania tarentolae RNA ligase.

In one aspect of the present invention, there is provided a manufacturing method for a single-stranded RNA according the manufacturing method as described above, wherein the RNA ligase is an RNA ligase consisting of an amino acid sequence having an identity of 95% or more with an amino acid sequence set forth in SEQ ID NO: 21, 22, or 23.

In one aspect of the present invention, there is provided a manufacturing method for a single-stranded RNA according to the manufacturing method as described above, wherein the RNA ligase is a T4 bacteriophage-derived T4 RNA ligase 2 or a KVP40-derived RNA ligase 2.

[Advantageous Effects of Invention]

According to the manufacturing method of the present invention, a single-stranded RNA can be simply manufactured.

[Brief Description of Drawings]

Fig. 1 is a schematic diagram showing an example of a bonding order of each region of first and second single-stranded RNAs.

Fig. 2 is a schematic diagram showing an example of a step for manufacturing a linked single-stranded RNA.

Fig. 3 is a schematic diagram showing another example of the bonding order of each region of the first and second single-stranded RNAs.

Fig. 4 is a schematic diagram showing another example of the step for manufacturing the linked single-stranded RNA.

Fig. 5 shows first and second single-stranded RNAs used in Example 1 and a generated linked single-stranded RNA.

Fig. 6 shows first and second single-stranded RNAs used in Example 2 and a generated linked single-stranded RNA.

[Description of Embodiments]

A manufacturing method according to one embodiment of the present invention is a manufacturing method for a single-stranded RNA, including a step of causing an RNA ligase which is classified as EC 6.5.1.3 defined by the International Union of Biochemistry and Molecular Biology as an enzyme number and has a double strand nick repair activity to act on a first single-stranded RNA having a phosphate group at the 5' end and a second single-stranded RNA having a hydroxyl group at the 3' end to link the first single-stranded RNA and the second single-stranded RNA. The linked single-stranded RNA obtained by the manufacturing method according to the present embodiment is a single-stranded RNA consisting of an X2 region, a Y2 region, a Ly linker region, a Y1 region, an X1 region, and a Z region from the 5' end. The Z region may consist of a Z1 region, a linker region Lz, and a region Z2 from the 5' end, and the linked single-stranded RNA may be a single-stranded RNA consisting of the X2 region, the Y2 region, the Ly linker region, the Y1 region, the X1 region, the Z1 region, the Lz linker region, and the region Z2. In the linked single-stranded RNA, at least one of a W1 region consisting of the Y1 region, the X1 region, and the Z region (or the Z1 region) and a W2 region consisting of the X2 region and the Y2 region may have a sequence that suppresses expression of a gene targeted by an RNA interference method.

Any one or both of the W1 region and the W2 region may have two or more identical expression suppression sequences for the same target gene, may have two or more different expression suppression sequences for the same target, or may have two or more different expression suppression sequences for different target genes. When the W1 region has two or more expression suppression sequences, an arrangement position of each expression suppression sequence is not particularly limited, and may be any one or both of the X1 region and the Y1 region or may be a region spanning the both. When the W2 region has two or more expression suppression sequences, an arrangement position of each expression suppression sequence may be any one or both of the X2 region and the Y2 region or may be a region spanning the both. The expression suppression sequence has a complementarity of preferably 90% or more, more preferably 95%, still more preferably 98%, and particularly preferably 100% with respect to a predetermined region of the target gene.

Such a linked single-stranded RNA is manufactured through a step of causing a ligase to act on the first single-stranded RNA having a phosphate group at the 5' end and the second single-stranded RNA having a hydroxyl group at the 3' end to link the first single-stranded RNA and the second single-stranded RNA (see Figs. 1 to 4).

In the linked single-stranded RNA, sequence portions having a complementarity are arranged in a molecule and a double strand can be partially formed in the molecule. As shown in Fig. 2, in a linked single-stranded RNA molecule, the X1 region and the X2 region comprise nucleotide sequences complementary to each other, the Y1 region and the Y2 region comprise nucleotide sequences complementary to each other, a double strand is formed between these sequences having a complementarity, and the Ly and Lz linker regions take loop structures according to a length thereof. Figs. 2 and 4 merely show a linking order of the regions and a positional relationship between the respective regions forming a double-stranded portion, and for example, a length of each region, a shape of the linker region (Ly and Lz), and the like are not limited thereto.

At least one of the W1 region consisting of the Y1 region, the X1 region, and the Z region (or the Z1 region) and the W2 region consisting of the X2 region and the Y2 region may have at least one sequence that suppresses expression of a gene targeted by the RNA interference method. In the linked single-stranded RNA, the W1 region and the W2 region may be completely complementary or may be non-complementary in one or several nucleotides, but are preferably completely complementary. The one or several nucleotides are, for example, one to three nucleotides and preferably one or two nucleotides. The Y1 region has a nucleotide sequence complementary to the entire region of the Y2 region. The Y1 region and the Y2 region have nucleotide sequences which are completely complementary to each other, and are nucleotide sequences having the same number of nucleotides of two or more. The X1 region has a nucleotide sequence complementary to the entire region of the X2 region. The X1 region and the X2 region have nucleotide sequences which are completely complementary to each other, and are nucleotide sequences having the same number of nucleotides of two or more. In the manufacturing method according to the present embodiment, the Z region is a region having a nucleotide sequence including any number of nucleotides, is not an essential sequence, and may adopt an aspect in which the number of nucleotides is 0 or an aspect in which one or more nucleotides are included. The Z region may be a region in which Z1, Lz, and Z2 regions are linked from the 5' end.

The length of each region is exemplified below, but is not limited thereto. In the present specification, a numerical range of the number of nucleotides indicates, for example, all positive integers belonging to the range, and as a specific example, a description of "one to four nucleotides" means indication of all of "one nucleotide", "two nucleotides", "three nucleotides", and "four nucleotides" (the same applies hereinafter).

In the linked single-stranded RNA molecule, a relationship among the number (W2n) of nucleotides in the W2 region, the number (X2n) of nucleotides in the X2 region, and the number (Y2n) of nucleotides in the Y2 region satisfies, for example, a condition of Expression (1). A relationship among the number (W1n) of nucleotides in the W1 region, the number (X1n) of nucleotides in the X1 region, and the number (Y1n) of nucleotides in the Y1 region satisfies, for example,

a condition of Expression (2).

$$W2n = X2n + Y2n \cdots (1)$$

$$W1n \geq X1n + Y1n \cdots (2)$$

In the linked single-stranded RNA molecule, a relationship between the number (X1n) of nucleotides in the X1 region and the number (Y1n) of nucleotides in the Y1 region is not particularly limited, and satisfies, for example, any one condition of the following expressions.

$$X1n = Y1n \cdots (3)$$

$$X1n < Y1n \cdots (4)$$

$$X1n > Y1n \cdots (5)$$

In the method according to the present embodiment, the number (X1n) of nucleotides in the X1 region, that is, the number (X2n) of nucleotides in the X2 region is 2 or more, preferably 4 or more, and more preferably 10 or more.

The number (Y1n) of nucleotides in the Y1 region that is, the number (Y2n) of nucleotides in the Y2 region is 2 or more, preferably 3 or more, and more preferably 4 or more.

The Z1 region preferably has a nucleotide sequence complementary to the entire region of the Z2 region or a partial region of the Z2 region. The Z1 region and the Z2 region may be non-complementary in one or several nucleotides, but are preferably completely complementary.

More specifically, the Z2 region preferably consists of a nucleotide sequence shorter than that of the Z1 region by one or more nucleotides. In this case, the entire nucleotide sequence of the Z2 region is complementary to all nucleotides in any partial region of the Z1 region. The nucleotide sequence from the 5' end to the 3' end of the Z2 region is more preferably a sequence which is complementary to a nucleotide sequence starting from a nucleotide at the 3' end of the Z1 region toward the 5' end.

In the linked single-stranded RNA molecule, the relationship between the number (X1n) of nucleotides in the X1 region and the number (X2n) of bases in the X2 region, the relationship between the number (Y1n) of nucleotides in the Y1 region and the number (Y2n) of nucleotides in the Y2 region, and the relationship between the number (Z1n) of nucleotides in the Z1 region and the number (Z2n) of nucleotides in the Z2 region satisfy conditions of Expressions (6), (7), and (8), respectively.

$$X1n = X2n \cdots (6)$$

$$Y1n = Y2n \cdots (7)$$

$$Z1n \geq Z2n \cdots (8)$$

A total length (total number of nucleotides) of the linked single-stranded RNA is not particularly limited. In the linked single-stranded RNA, the lower limit of the sum of the number of nucleotides (the number of nucleotides in the total length) is typically 38, preferably 42, more preferably 50, still more preferably 51, and particularly preferably 52, and the upper limit thereof is typically 300, preferably 200, more preferably 150, still more preferably 100, and particularly preferably 80. In the linked single-stranded RNA, the lower limit of the sum of the number of nucleotides excluding the linker region (Ly and Lz) is typically 38, preferably 42, more preferably 50, still more preferably 51, and particularly preferably 52, and the upper limit thereof is typically 300, preferably 200, more preferably 150, still more preferably 100, and particularly preferably 80.

In the linked single-stranded RNA, the lengths of the Ly and Lz linker regions are not particularly limited. For

example, these linker regions preferably have such a length that the X1 region and the X2 region can form a double strand, or such a length that the Y1 region and the Y2 region can form a double strand. Each of the linker regions Ly and Lz is a region having an atomic group derived from an amino acid. These linker regions (Ly and Lz) are usually non-nucleotide linker regions.

The upper limit of the number of atoms forming a main chain of the linker region is typically 100, preferably 80, and more preferably 50.

The Ly linker region is, for example, a divalent group represented by Formula (1), and Lz is, for example, a divalent group represented by Formula (1').

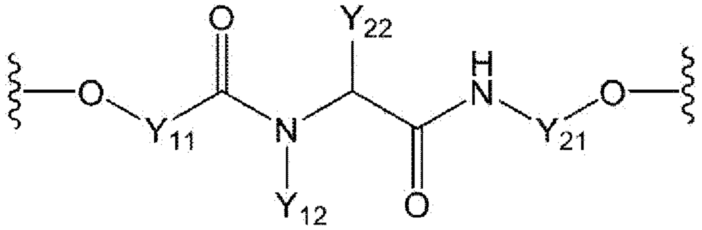

(I)

In the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms; a terminal oxygen atom bonded to $Y_{11}$ is bonded to a phosphorus atom of a phosphate ester of a terminal nucleotide in any one region of the Y1 region and the Y2 region; and a terminal oxygen atom bonded to $Y_{21}$ is bonded to a phosphorus atom of a phosphate ester of a terminal nucleotide in the other region of the Y1 region and the Y2 region, which is not bonded to $Y_{11}$.

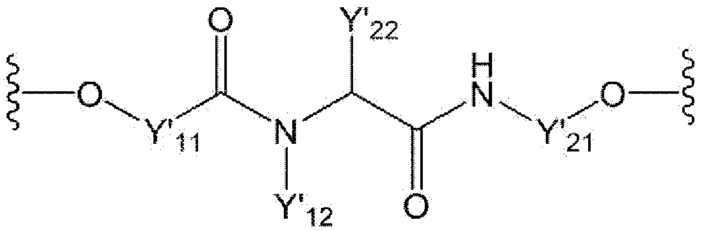

(I')

In the formula, $Y'_{11}$ and $Y'_{21}$ each independently represent an alkylene group having 1 to 20 carbon atoms, and $Y'_{12}$ and $Y'_{22}$ each independently represent a hydrogen atom or an alkyl group which may be substituted with an amino group, or $Y'_{12}$ and $Y'_{22}$ are bonded to each other at their terminals to represent an alkylene group having 3 or 4 carbon atoms; a terminal oxygen atom bonded to $Y'_{11}$ is bonded to a phosphorus atom of a phosphate ester of a terminal nucleotide in any one region of the Z1 region and the Z2 region; and a terminal oxygen atom bonded to $Y'_{21}$ is bonded to a phosphorus atom of a phosphate ester of a terminal nucleotide in the other region of the Z1 region and the Z2 region, which is not bonded to $Y'_{11}$.

The alkylene group having 1 to 20 carbon atoms as $Y_{11}$ and $Y_{21}$, and $Y'_{11}$ and $Y'_{21}$ preferably has 1 to 10 carbon atoms and more preferably has 1 to 5 carbon atoms. The alkylene group may be linear or branched.

The alkyl group which may be substituted with an amino group as $Y_{12}$ and $Y_{22}$, and $Y'_{12}$ and $Y'_{22}$ preferably has 1 to 10 carbon atoms and more preferably has 1 to 5 carbon atoms. The alkyl group may be linear or branched.

Suitable examples of a Ly linker and a Lz linker include a divalent group having a structure represented by Formula (II-A) or (II-B).

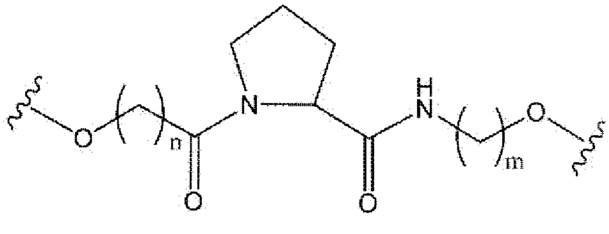

(II-A)

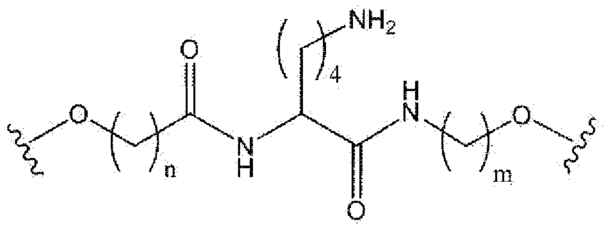

(II-B)

In the formulae, n and m each independently represent any integer of 1 to 20.

n and m are preferably any integer of 1 to 10 and more preferably any integer of 1 to 5.

In a preferred aspect, the Ly linker and the Lz linker each independently represent a divalent group represented by either Formula (II-A) or (II-B).

Specific examples of the first single-stranded RNA and the second single-stranded RNA include single-stranded RNAs described in Figs. 5 and 6.

In a link reaction carried out by the manufacturing method according to the present embodiment, an RNA ligase (hereinafter, also referred to as "the present RNA ligase" in some cases) which is classified as EC 6.5.1.3 defined by the International Union of Biochemistry and Molecular Biology as an enzyme number and has a double strand nick repair activity is used. Examples of such an RNA ligase include a T4 bacteriophage-derived T4 RNA ligase 2. The RNA ligase 2 can be purchased from, for example, (New England Biolabs). Furthermore, examples of the RNA ligase include a vibriophage KVP40-derived ligase 2, a Trypanosoma brucei RNA ligase, a Deinococcus radiodurans RNA ligase, or a Leishmania tarentolae RNA ligase. As such an RNA ligase, for example, a ligase obtained by performing extraction and purification from each organism by the method described in Non Patent Literature (Structure and Mechanism of RNA Ligase, Structure, Vol. 12, PP. 327 to 339) can also be used.

As the T4 bacteriophage-derived T4 RNA ligase 2, an RNA ligase which is a protein consisting of an amino acid sequence having an identity of 95% or more with an amino acid sequence set forth in SEQ ID NO: 21 and has a double strand nick repair activity can also be used. As such an RNA ligase 2, in addition to an enzyme having the amino acid sequence set forth in SEQ ID NO: 21, T39A, F65A, and F66A (see RNA ligase structures reveal the basis for RNA specificity and conformational changes that drive ligation forward, Cell. Vol. 127, pp.71 to 84), which are mutants thereof, can be exemplified. Such an RNA ligase 2 can be obtained, for example, by a method using Escherichia coli bacteriophage T4 which is deposited with the American Type Culture Collection (ATCC) as ATCC (registered trademark) 11303, based on the description in the above-mentioned literature, or a method such as PCR.

The KVP40-derived RNA ligase 2 can be obtained by the method described in Non Patent Literature (Characterization of bacteriophage KVP40 and T4 RNA ligase 2, Virology, vol. 319, PP. 141 to 151). Specifically, for example, the KVP40-derived RNA ligase 2 can be obtained by the following method. That is, in DNAs extracted from bacteriophage KVP40 (for example, deposited with JGI as an accession number of Go008199), an open reading frame 293 is amplified by a polymerase chain reaction after restriction enzyme digestion with Ndel and BamHI. The obtained DNA is incorporated into a plasmid vector pET16b (Novagen). Alternatively, the DNA sequence can be artificially synthesized by PCR. Here, a desired mutant can be obtained by DNA sequence analysis. Subsequently, the obtained vector DNA is incorporated into E. coli BL21(DE3), and the resultant is cultured in a LB medium containing 0.1 mg/mL ampicillin. Isopropyl-β-thiogalactoside is added thereto so that a concentration is 0.5 mM, and cultured at 37°C for 3 hours. All subsequent operations are preferably performed at 4°C. First, bacterial cells are precipitated by a centrifugal operation, and the precipitate is stored at -80°C. A buffer A [50 mM Tris-HCl (pH of 7.5), 0.2 M NaCl, and 10% sucrose] is added to the frozen bacterial cells. Then, lysozyme and Triton X-100 are added thereto, and the bacterial cells are disrupted by ultrasonic waves to elute a target substance. Thereafter, the target substance is isolated by using affinity chromatography, size exclusion chromatography, or the like. Then, the obtained aqueous solution is subjected to centrifugal filtration, and the resultant can be used as a ligase by replacing an eluate with a buffer.

In this way, the KVP40-derived RNA ligase 2 can be obtained. As the KVP40-derived RNA ligase 2, an RNA ligase which is a protein consisting of an amino acid sequence having an identity of 95% or more with an amino acid sequence of SEQ ID NO: 22 and has a double strand nick repair activity can be used.

The Deinococcus radiodurans RNA ligase can be obtained by the method described in Non Patent Literature (An RNA Ligase from Deinococcus radiodurans, J Biol Chem., Vol. 279, No. 49, PP. 50654 to 61). For example, the ligase can also be obtained from a biological material which is deposited with the ATCC as ATCC (registered trademark) BAA-816. As the Deinococcus radiodurans RNA ligase, an RNA ligase which is a protein consisting of an amino acid sequence having an identity of 95% or more with an amino acid sequence of SEQ ID NO: 23 and has a double strand nick repair activity can be used. Specific examples of such a ligase include an RNA ligase consisting of an amino acid sequence having a mutation of K165A or E278A in the RNA ligase of SEQ ID NO: 23, in addition to the RNA ligase consisting of the amino acid sequence of SEQ ID NO: 23 (An RNA Ligase from Deinococcus radiodurans, J Biol Chem.,

Vol. 279, No. 49, PP. 50654 to 61).

The Trypanosoma brucei RNA ligase can be obtained by the method described in Non Patent Literature (Assiciation of Two Novel Proteins TbMP52 and TbMP48 with the Trypanosoma brucei RNA Editing Complex, Vol. 21, No. 2, PP. 380 to 389).

The Leishmania tarentolae RNA ligase can be obtained by the method described in Non Patent Literature (The Mitochondrial RNA Ligase from Leishmania tarentolae Can Join RNA Molecules Bridged by a Complementary RNA, Vol. 274, No. 34, PP. 24289 to 24296).

A reaction condition for the manufacturing method according to the present embodiment using the present RNA ligase is not particularly limited as long as the present RNA ligase functions under the condition, but as one typical example, a condition in which a first nucleic acid strand, a second nucleic acid strand, a Tris-HCl buffer solution (pH of 7.5) containing ATP, magnesium chloride, and DTT, and pure water are mixed, the present RNA ligase is added to the liquid mixture, and then the resultant is reacted at a temperature (for example, 37°C) at which the ligase functions for a predetermined time (for example, 1 hour) is exemplified.

Alternatively, the manufacturing method according to the present embodiment can also be performed according to the conditions described in Non Patent Literature (Bacteriophage T4 RNA ligase 2 (gp24-1) exemplifies a family of RNA ligases found in all, Proc. Natl. Acad. Sci, 2002, Vol. 99, No. 20, PP. 12709 to 12714).

A crude product obtained by the link reaction of the first single-stranded RNA and the second single-stranded RNA using the present RNA ligase can usually be isolated by using a method for precipitating, extracting, and purifying the RNA.

Specifically, a method for precipitating an RNA by adding a solvent having low solubility to the RNA, such as ethanol or isopropyl alcohol, to a solution after a link reaction, or a method for adding a mixed solution of phenol/chloroform/isoamyl alcohol (for example, phenol/chloroform/isoamyl alcohol = 25/24/1) to a solution after a link reaction and extracting an RNA into an aqueous layer is employed.

Examples of a method for purifying the linked RNA include known methods such as reverse-phase column chromatography, anion-exchange column chromatography, affinity column chromatography, and gel filtration column chromatography, and the linked RNA can be isolated from the crude product by using an appropriate combination of these methods.

The first single-stranded RNA can be prepared, for example, by a solid-phase synthesis method. More specifically, the first single-stranded RNA can be prepared using a nucleic acid synthesizer (NTS M-4MX-E (manufactured by NIHON TECHNO SERVICE CO., LTD.)) based on a phosphoramidite method. The phosphoramidite method is a method in which three steps of deblocking, coupling, and oxidation are set as one cycle, and the cycle is repeated until a desired base sequence is obtained. Regarding each reagent, for example, the preparation can be performed by using porous glass as a solid-phase carrier, using a dichloroacetic acid-toluene solution as a deblocking solution, using 5-benzylmercapto-1H-tetrazole as a coupling agent, using an iodine solution as an oxidizing agent, and using an acetic anhydride solution and an N-methylimidazole solution as a capping solution. Excision from the solid-phase carrier after solid-phase synthesis and deprotection can be performed, for example, according to the method described in PCT International Publication No. WO2013/027843. That is, after deprotection of a base moiety and a phosphate group and excision thereof from the solid-phase carrier are performed by adding an ammonia aqueous solution and ethanol, the solid-phase carrier is filtered, then deprotection of a 2'-hydroxyl group is performed using tetrabutylammonium fluoride, and thus an RNA can be prepared.

An amidite used in such a solid-phase synthesis method is not particularly limited, and for example, a TBDMS amidite (TBDMS RNA Amidites, trade name, ChemGenes Corporation), an ACE amidite, a TOM amidite, a CEE amidite, a CEM amidite, and a TEM amidite (general remarks of Chakhmakhcheva: Protective Groups in the Chemical Synthesis of Oligoribonucleotides, Russian Journal of Bioorganic Chemistry, 2013, Vol. 39, No. 1, pp. 1 to 21) EMM amidite (described in PCT International Publication No. WO2013/027843), in which $R_1$ in Structural Formula (III-a) is protected with a tertiary butyldimethylsilyl (TBDMS) group, a bis(2-acetoxy) methyl (ACE) group, a (triisopropylsilyloxy) methyl (TOM) group, a (2-cyanoethoxy) ethyl (CEE) group, a (2-cyanoethoxy) methyl (CEM) group, a para-toluylsulfonylethoxymethyl (TEM) group, or a (2-cyanoethoxy) methoxymethyl (EMM) group, can also be used.

In addition, regarding the Ly linker region and the Lz linker region, an amidite having a proline skeleton, which is represented by Structural Formula (III-b), can be used in the method of Example A4 in PCT International Publication No. WO2012/017919. Moreover, by using an amidite (see Examples A1 to A3 in PCT International Publication No. WO2013/103146) represented by any one of Structural Formulae (III-c), (III-d), and (III-e), the linker regions can be similarly prepared using the nucleic acid synthesizer.

For phosphorylation at the 5'-position of the 5' end, an amidite for phosphorylation at the 5' end may be used in solid-phase synthesis. A commercially available amidite can be used as the amidite for phosphorylation at the 5' end. Moreover, in the solid-phase synthesis, an RNA molecule having a hydroxyl group or a protected hydroxyl group at the 5'-position of the 5' end is synthesized, deprotection is appropriately performed, then phosphorylation is performed with a commercially available phosphorylating agent, and thus a single-stranded RNA having a phosphate group at the 5'

end can be prepared. As the phosphorylating agent, a commercially available Chemical Phosphorylation Reagent (Glen Research) represented by Structural Formula (III-f) is known (Patent Literature EP0816368).

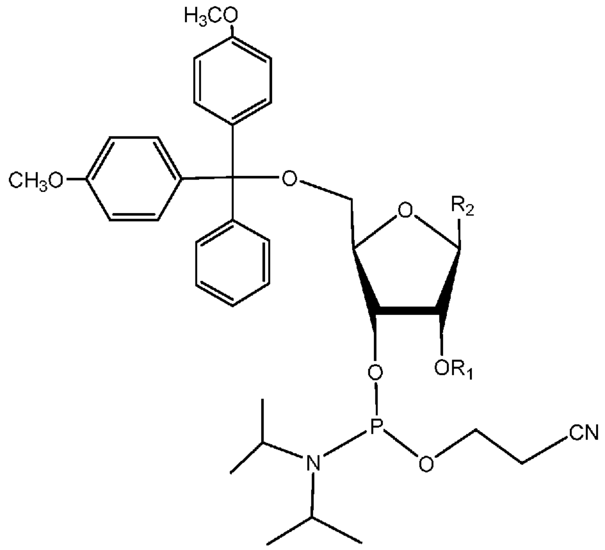

(IIIa)

In Formula (III-a), $R_2$ represents a nucleic acid base which may be protected with a protective group, and $R_1$ represents a protective group.

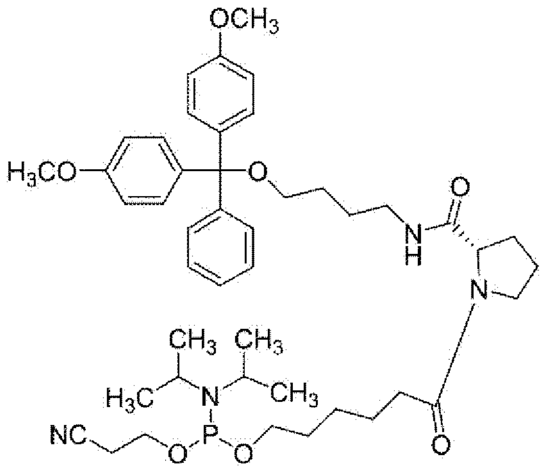

(III-b)

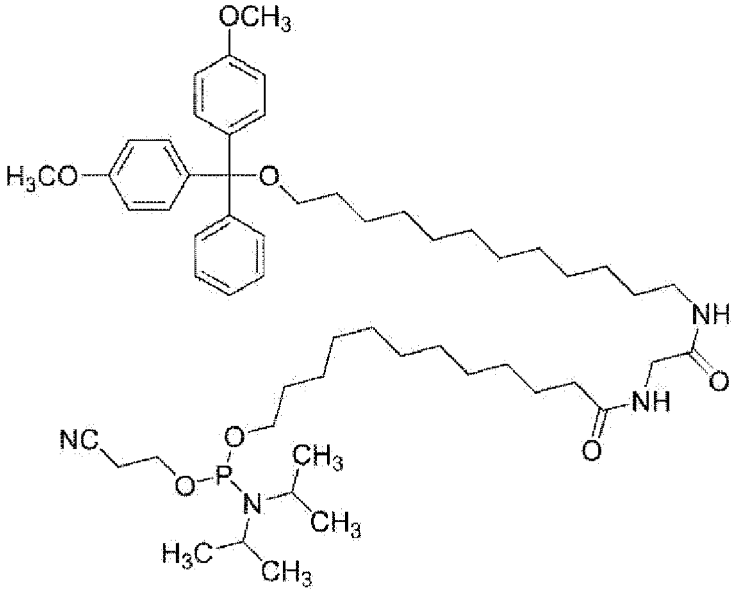

(III-c)

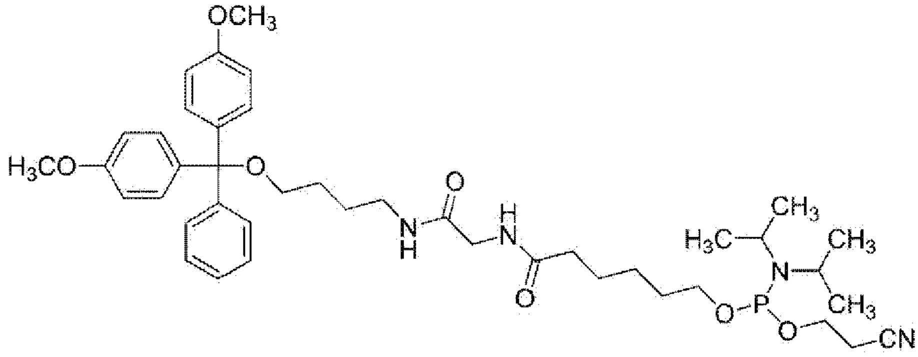

(III-d)

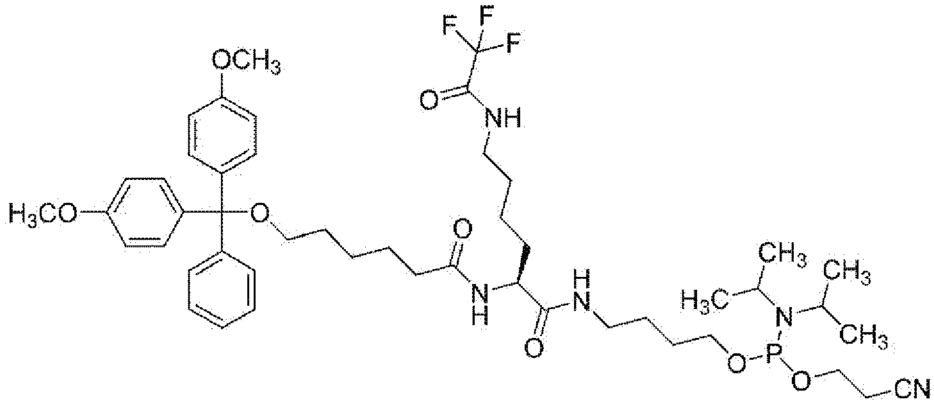

(III-e)

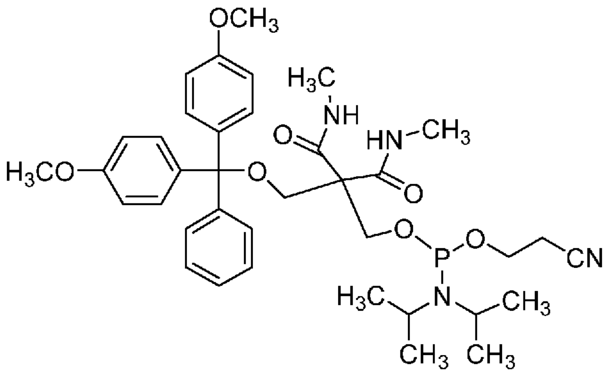

(III-f)

The second single-stranded RNA can be similarly manufactured using the nucleic acid synthesizer based on the solid-phase synthesis method, that is, the phosphoramidite method.

Bases constituting a nucleotide are usually natural bases constituting a nucleic acid, typically an RNA, but unnatural bases may be used in some cases. Examples of such an unnatural base include modified analogs of natural or unnatural bases.

Examples of a base include a purine base such as adenine and guanine, and a pyrimidine base such as cytosine, uracil, and thymine. As the base, in addition to the above bases, inosine, xanthine, hypoxanthine, nubularine, isoguanisine, tubercidine, and the like are exemplified. Examples of the bases include an alkyl derivative such as 2-aminoadenine and 6-methylated purine; an alkyl derivative such as 2-propylated purine; 5-halouracil and 5-halocytosine; 5-propynyluracil and 5-propynylcytosine; 6-azouracil, 6-azocytosine, and 6-azothymine; 5-uracil (pseudouracil), 4-thiouracil, 5-halouracil, 5-(2-aminopropyl) uracil, 5-aminoallyluracil; 8-halo, aminated, thiolated, thioalkylated, hydroxylated, or other 8-substituted purine; 5-trifluoromethylated or other 5-substituted pyrimidine; 7-methylguanine; 5-substituted pyrimidine; 6-azapyrimidine; N-2-, N-6-, or O-6-substituted purine (including 2-aminopropyl adenine); 5-propynyluracil and 5-propynylcytosine; dihydrouracil; 3-deaza-5-azacytosine; 2-aminopurine; 5-alkyluracil; 7-alkylguanine; 5-alkylcytosine; 7-deazaadenine; N6,N6-dimethyladenine; 2,6-diaminopurine; 5-amino-allyl-uracil; N3-methyluracil; substituted 1,2,4-triazole; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl) uracil; 3-methylcytosine; 5-methylcytosine; N4-acetylcytosine; 2-thiocytosine; N6-methyladenine; N6-isopentyladenine; 2-methylthio-N6-isopentenyladenine; N-methylguanine; and an O-alkylated base. Moreover, examples of the purine base and the pyrimidine base include the bases disclosed in United States Patent No. 3687808, "Concise Encyclopedia Of Polymer Science And Engineering", pp. 858 and 859, edited by Kroschwitz J. I., John Wiley & Sons, 1990, and Englisch et al., Angewandte Chemie, International Edition, 1991, vol. 30, p. 613.

A single-stranded RNA nucleic acid molecule which is obtained by the method according to the present embodiment and consists of the X1 region, the Y1 region, the Ly region, the Y2 region, the X2 region, and the Z region from the 5' end has the 5' end and the 3' end which are not linked to each other, and can also be referred to as a linear single-stranded nucleic acid molecule. Such a single-stranded RNA nucleic acid molecule can be used for suppressing expression of a target gene, for example, in vivo or in vitro, and can be used for suppressing the expression of the target gene by RNA interference. "Suppressing the expression of the target gene" means, for example, inhibition of expression of a target gene. A mechanism of the suppression is not particularly limited, and may be, for example, down regulation or silencing.

The suppression of the expression of the target gene can be confirmed, for example, by a decrease in an amount of a transcriptional product generated from the target gene, a decrease in an activity of the transcriptional product, a decrease in an amount of a translation product generated from the target gene, or a decrease in an activity of the translation product. Examples of a protein as the translation product include a mature protein and a precursor protein before undergoing processing or post-translational modification.

[Examples]

Hereinafter, Examples according to the present embodiment will be described in detail, but the present invention is not limited thereto.

[Example 1]

1. Synthesis of first single-stranded RNA

As an RNA of a strand I in Example 1, the following single-stranded RNA (Sequence 1 in Table 2; SEQ ID NO: 1) was synthesized. The strand I has a length of 13 bases and corresponds to the first single-stranded RNA.
Strand I (Sequence 1): 5'-pGUGUACUCUGCUU-3'

The single-stranded RNA was synthesized from a 3' side to a 5' side using a nucleic acid synthesizer (trade name NTS M-4MX-E, NIHON TECHNO SERVICE CO., LTD.) based on a phosphoramidite method. The synthesis was performed by using the uridine EMM amidite described in Example 2, the cytidine EMM amidite described in Example 3, the adenosine EMM amidite described in Example 4, and the guanosine EMM amidite described in Example 5 in PCT International Publication No. WO2013/027843 and a Chemical Phosphorylation Reagent (Glen Research) for 5' phosphorylation as an RNA amidite, using porous glass as a solid-phase carrier, using a high-purity trichloroacetic acid-toluene solution as a deblocking solution, using 5-benzylmercapto-1H-tetrazole as a condensing agent, using an iodine solution as an oxidizing agent, and using a phenoxyacetic acid solution and an N-methylimidazole solution as a capping solution. Excision from the solid-phase carrier after solid-phase synthesis and deprotection followed the method described in PCT International Publication No. WO2013/027843. That is, after an ammonia aqueous solution and ethanol were added and the mixture was allowed to stand for a while, the solid-phase carrier was filtered and then deprotection of a hydroxyl group was performed using tetrabutylammonium fluoride. The obtained RNA was dissolved in distilled water for injection so as to have a desired concentration.

2. Synthesis of second single-stranded RNA

As an RNA of a strand II in Example 1, the following single-stranded RNA (Sequence 2 in Table 2; SEQ ID NOs: 2 and 3) was synthesized. The strand II has a length of 36 bases and corresponds to the second single-stranded RNA.
Strand II (Sequence 2): 5'-GCAGAGUACACACAGCAUAUACCKGGUAUAUGCUGU-3'

The single-stranded RNA was synthesized using the nucleic acid synthesizer (trade name NTS M-4MX-E, NIHON TECHNO SERVICE CO., LTD.) based on the phosphoramidite method. In the synthesis, the uridine EMM amidite described in Example 2, the cytidine EMM amidite described in Example 3, the adenosine EMM amidite described in Example 4, and the guanosine EMM amidite described in Example 5 in PCT International Publication No. WO2013/027843 and the lysine amidite (Ie) described in Example A3 in PCT International Publication No. WO2012/017919 were used as an RNA amidite. Excision from the solid-phase carrier after solid-phase synthesis and deprotection followed the method described in PCT International Publication No. WO2013/027843. The obtained RNA was dissolved in distilled water for injection so as to have a desired concentration.

3. Ligation reaction

Next, a ligation reaction between the first RNA strand and the second RNA strand was performed at a reaction scale of 7.9 mL with a composition of a 160 units/mL T4 RNA ligase 2, 50 mM Tris-HCl (pH of 7.5), 2 mM $MgCl_2$, 1 mM DTT, and 400 μM ATP.

The obtained linked single-stranded RNA is shown below (Sequence 15; SEQ ID NOs: 17 and 18).

5'-

GCAGAGUACACACAGCAUAUACCKGGUAUAUGCUGUGUGUACUCUGCUU-

3'

Specifically, first, the first RNA strand and the second RNA strand were added into a 15-mL tube, which contains 790 μL of a 4000 μM ATP buffer solution (10× buffer solution) containing 500 mM Tris-HCl (pH of 7.5), 20 mM $MgCl_2$, and 10 mM DTT and distilled water for injection, so that a concentration of the first RNA strand was 5.5 μM and a concentration of the second RNA strand was 5.0 μM, and the mixture was allowed to stand in a 65°C water bath for 10 minutes. Thereafter, the T4 RNA ligase 2 (manufactured by New England Biolabs) was added so that the above composition and the above reaction scale were satisfied, and the mixture was incubated at 25°C for 24 hours. After the reaction, a reaction solution was purified by column chromatography under conditions shown in Table 1 below. The obtained fractions were each analyzed by HPLC. Fractions having a purity of 90% or more were collected and mixed.

As a result, a target substance could be obtained with a purity of 90% and a total yield of 11%. Here, the total yield is a proportion of an amount of a single-stranded RNA obtained by purifying a product after a ligation reaction with respect to an amount of an RNA strand charged. The amount of the RNA is determined based on an absorbance of ultraviolet rays having a wavelength of 260 nm. The amount is a value obtained by assuming that a concentration of the RNA when the absorbance is 1 is 33 μg/mL. As a result of measuring the obtained sample by mass spectrometry measurement, the measured value matched a calculated value, and thus it can be confirmed that the target substance was obtained (measured value: 15715.2464 and theoretical value: 15715.2075).

[Table 1]

| | | | | | |
|---|---|---|---|---|---|
| Detector | UV 260 nm | | | | |
| Column | Triart C18 manufactured by YMC CO., LTD., 150 mm × 4.6 mm ID | | | | |
| Column temperature | 60 °C | | | | |
| Flow rate | 1.0 mL/min | | | | |
| Mobile phase A | 100 mM dibutylammonium acetate (pH of 7.0) | | | | |
| Mobile phase B | Acetonitrile | | | | |
| Fractionation width | 1.25 mL | | | | |
| Gradient conditions | Time (min) | 0 | 5 | 45 | 50 |
| | Concentration (%) of mobile phase B | 10 | 35 | 43 | 100 |

[Example 2]

(Synthesis of linked single-stranded RNA in which Ly and Lz contain proline derivative)

The obtained linked single-stranded RNA is shown below (Sequence 16; SEQ ID NOs: 19 and 20).

5'-

AGCAGAGUACACACAGCAUAUACCPGGUAUAUGCUGUGUGUACUCUGCUU

CPG-3'

1. Synthesis of first single-stranded RNA

As an RNA of a strand I in Example 2, a single-stranded RNA (Sequence 3 in Table 2; SEQ ID NO: 4) of a strand I (Sequence 3) shown in Table 3 was synthesized. The strand I has a length of 16 bases and corresponds to the first single-stranded RNA.

The single-stranded RNA was synthesized using the nucleic acid synthesizer (trade name NTS M-4MX-E, NIHON TECHNO SERVICE CO., LTD.) based on the phosphoramidite method. In the synthesis, the uridine EMM amidite described in Example 2, the cytidine EMM amidite described in Example 3, the adenosine EMM amidite described in Example 4, and the guanosine EMM amidite described in Example 5 in PCT International Publication No. WO2013/027843 and the proline amidite (Ib) described in Example A3 in PCT International Publication No. WO2012/017919 were used as an RNA amidite. For 5' phosphorylation, the Chemical Phosphorylation Reagent (Glen Research) was used (the same applies hereinafter). Excision from the solid-phase carrier after solid-phase synthesis and deprotection followed the method described in PCT International Publication No. WO2013/027843. The obtained RNA was dissolved using distilled water for injection so as to have a desired concentration.

2. Synthesis of second RNA

As an RNA of a strand II in Example 2, a single-stranded RNA (Sequence 4 in Table 2; SEQ ID NOs: 5 and 6) of a strand II (Sequence 4) shown in Table 3 was synthesized. The strand II has a length of 37 bases and corresponds to the second single-stranded RNA.

Operations after deprotection in the synthesis of the second single-stranded RNA strand were performed in

the same manner as in the synthesis of the first single-stranded RNA.

3. Ligation reaction

Next, a ligation reaction between the first RNA strand and the second RNA strand was performed at a reaction scale of 10 mL with a composition of a 58 units/mL T4 RNA ligase 2, 50 mM Tris-HCl (pH of 7.5), 2 mM $MgCl_2$, 1 mM DTT, and 400 $\mu$M ATP.

Specifically, first, the first RNA strand and the second RNA strand were added to a solution, which contains 0.96 mL of a 4000 $\mu$M ATP buffer solution (10× buffer solution) containing 500 mM Tris-HCl (pH of 7.5), 20 mM $MgCl_2$, and 10 mM DTT and distilled water for injection, so that the concentration of the first RNA strand was 4.4 $\mu$M and the concentration of the second RNA strand was 4.0 $\mu$M, and the mixture was allowed to stand in a 65°C water bath for 10 minutes. Thereafter, the T4 RNA ligase 2 (New England Biolabs) was added so that the above composition and the above reaction scale were satisfied, and the mixture was incubated at 25°C for 24 hours. After the reaction, a reaction solution was purified by column chromatography under conditions shown in Table 1. The obtained fractions were analyzed by HPLC, and fractions (fractions in which a proportion occupied by a peak area of the target substance with respect to a total area of peaks detected in a chromatogram was 90% or more) having a purity of 90% or more were collected and mixed. As a result, a target substance could be obtained with a purity of 94% and a total yield of 14%. Furthermore, as a result of measuring the obtained sample by mass spectrometry measurement, the measured value matched a calculated value, and thus it can be confirmed that the target substance was obtained (measured value: 17025.4858 and theoretical value: 17025.4672).

[Examples 3 to 6]

Hereinafter, in Examples 3 to 6, the reaction was performed using the T4 RNA ligase 2, a reaction product was purified by column chromatography, and the obtained fractions were analyzed by HPLC, in the same manner as in Example 2, except that the strand I (corresponding to the first single-stranded RNA) and the strand II (corresponding to the second single-stranded RNA) shown in Table 3 were each synthesized using the nucleic acid synthesizer and used. Regarding each of Examples 3 to 6, fractions (the same definition as above) having a purity of 90% or more were collected and mixed, and purities and total yields thereof were determined, respectively. The results are summarized in Table 2.

[Reference Example 1]

In order to synthesize the same linked single-stranded RNA (Sequence 16) as in Example 2, operations were performed in the same manner as in Example 2 except that the strand I (Sequence 13) and the strand II (Sequence 14) shown in Table 3 were synthesized using the nucleic acid synthesizer and used. At this time, the number of nucleotides in a region corresponding to the Y1 region in the strand II (Sequence 14) was 1. As a result of analyzing a reaction solution after a ligation reaction by HPLC, change was not observed in the peaks of the strand I and the strand II, a peak was not detected at an elution position of the linked single-stranded RNA, and thus it was confirmed that a link reaction did not proceed.

[Table 2]

| | RNA strand | Strand length | Number of nucleotides in each region | | | | | | | Linker | | Purity | Total yield |
|---|---|---|---|---|---|---|---|---|---|---|---|---|---|
| | | | X1 | X2 | Y1 | Y2 | Z | Z1 | Z2 | Ly | Lz | | |
| Example 1 | Strand I: Sequence 1 | 13 | 11 | - | - | - | 2 | - | - | - | - | 90% | 11% |
| | Strand II: Sequence 2 | 36 | | 11 | 12 | 12 | | | | K | | | |

(continued)

| | RNA strand | Strand length | Number of nucleotides in each region | | | | | | | Linker | | Purity | Total yield |
|---|---|---|---|---|---|---|---|---|---|---|---|---|---|
| | | | X1 | X2 | Y1 | Y2 | Z | Z1 | Z2 | Ly | Lz | | |
| Example 2 | Strand I: Sequence 3 | 16 | 12 | | | | | 2 | 1 | | P | 94% | 14% |
| | Strand II: Sequence 4 | 37 | - | 12 | 12 | 12 | - | - | - | P | - | | |
| Example 3 | Strand I: Sequence 5 | 20 | 16 | - | - | - | - | 2 | 1 | - | P | 95% | 12% |
| | Strand II: Sequence 6 | 33 | | 16 | 8 | 8 | | | | P | | | |
| Example 4 | Strand I: Sequence 7 | 22 | 18 | | | | | 2 | 1 | | P | 97% | 11% |
| | Strand II: Sequence 8 | 31 | | 18 | 6 | 6 | | | | P | | | |
| Example 5 | Strand I Sequence 9 | 24 | 20 | | | | | 2 | 1 | | P | 97% | 15% |
| | Strand II: Sequence 10 | 29 | - | 20 | 4 | 4 | - | - | - | P | - | | |
| Example 6 | Strand I: Sequence 11 | 26 | 22 | - | - | - | - | 2 | 1 | - | P | 98% | 11% |
| | Strand II: Sequence 12 | 27 | | 22 | 2 | 2 | | | | P | | | |
| Reference Example 1 | Strand I: Sequence 13 | 27 | 23 | | | | | 2 | 1 | | P | 0% | 0% |
| | Strand II: Sequence 14 | 26 | | 23 | 1 | 1 | | | | P | | | |

The sequences of the RNAs of the strands I and the strands II used in Examples and Reference Example are summarized in Table 3.

[Table 3]

| | Sequence (5' to 3') | |
|---|---|---|
| Strand I (Sequence 3) | pGUGUACUCUGCUUCPG | (SEQ ID NO: 4) |
| Strand II (Sequence 4) | AGCAGAGUACACACAGCAUAUACCPGGUAUAUGCUGU | (SEQ ID NOs: 5 and 6) |
| Strand I (Sequence 5) | pCUGUGUGUACUCUGCUUCPG | (SEQ ID NO: 7) |
| Strand II (Sequence 6) | AGCAGAGUACACACAGCAUAUACCPGGUAUAUG | (SEQ ID NO: 8) |

(continued)

| | Sequence (5' to 3') | |
|---|---|---|
| Strand I (Sequence 7) | pUGCUGUGUGUACUCUGCUUCPG | (SEQ ID NO: 9) |
| Strand II (Sequence 8) | AGCAGAGUACACACAGCAUAUACCPGGUAUA | (SEQ ID NO: 10) |
| Strand I (Sequence 9) | pUAUGCUGUGUGUACUCUGCUUCPG | (SEQ ID NO: 11) |
| Strand II (Sequence 10) | AGCAGAGUACACACAGCAUAUACCPGGUA | (SEQ ID NO: 12) |
| Strand I (Sequence 11) | pUAUAUGCUGUGUGUACUCUGCUUCPG | (SEQ ID NO: 13) |
| Strand II (Sequence 12) | AGCAGAGUACACACAGCAUAUACCPGG | (SEQ ID NO: 14) |
| Strand I (Sequence 13) | pGUAUAUGCUGUGUGUACUCUGCUUCPG | (SEQ ID NO: 15) |
| Strand II (Sequence 14) | AGCAGAGUACACACAGCAUAUACCPG | (SEQ ID NO: 16) |

[Example 7]

The strand I (Sequence 11) and the strand II (Sequence 12) shown in Table 3 were respectively used as the first RNA strand and the second RNA strand, and a ligation reaction between the first RNA strand and the second RNA strand was performed at a reaction scale of 15 mL with a composition of a 58 units/mL KVP40 RNA ligase 2, 50 mM Tris-HCl (pH of 7.0), 2 mM $MgCl_2$, 1 mM DTT, and 400 μM ATP.

The first RNA strand and the second RNA strand were added to a solution, which contains 166 μL of a 4000 μM ATP buffer solution (10× buffer solution) containing 500 mM Tris-HCl (pH of 7.0), 20 mM $MgCl_2$, and 10 mM DTT and distilled water for injection, so that the concentration of the first RNA strand (Sequence 11) was 2.6 μM and the concentration of the second RNA strand (Sequence 12) was 2.4 μM, and the mixture was allowed to stand in a 37°C water bath for 10 minutes. Thereafter, the KVP40 RNA ligase 2 (ProteinExpress Co., Ltd.) was added so that the above composition and the above reaction scale were satisfied, and the mixture was incubated at 25°C for 24 hours. After the reaction, 1 mL of a 0.2 M sodium ethylenediaminetetraacetate aqueous solution was added to a reaction solution, and the mixture was heated in a 65°C water bath for 10 minutes to inactivate an enzyme. The mixture as it is was analyzed by HPLC under the conditions in Table 1.

After the ligation reaction, the first single-stranded RNA and the second single-stranded RNA was decreased, and a new peak with a delayed retention time was observed. As a result of checking a molecular weight of the elution peak by mass spectrometry measurement, the molecular weight matched a calculated value of the linked single-stranded RNA, and thus it can be confirmed that a target linked single-stranded RNA was generated by the ligation reaction.

After the obtained fractions were subjected to HPLC analysis under the conditions shown in Table 1, fractions obtained with a purity of 95% or more were mixed. As a result, a target substance could be obtained with a purity of 98% and a total yield of 16%.

[Comparative Example 1]

As Comparative Example, a single-stranded RNA having the same nucleotide sequence as that of the linked single-stranded RNA (Sequence 16) synthesized in Example 2 was synthesized in the following manner from a 3' side to a 5' side using the nucleic acid synthesizer (trade name NTS M-4MX-E, NIHON TECHNO SERVICE CO., LTD.) based on the phosphoramidite method. The synthesis was performed by using the uridine EMM amidite described in Example 2, the cytidine EMM amidite described in Example 3, the adenosine EMM amidite described in Example 4, and the guanosine EMM amidite described in Example 5 in PCT International Publication No. WO2013/027843 and the Chemical Phosphorylation Reagent (Glen Research) for 5' phosphorylation as an RNA amidite, using porous glass as a solid-phase carrier, using a high-purity trichloroacetic acid-toluene solution as a deblocking solution, using 5-benzylmercapto-1H-tetrazole as a condensing agent, using an iodine solution as an oxidizing agent, and using a phenoxyacetic acid solution and an N-methylimidazole solution as a capping solution. Excision from the solid-phase carrier after solid-phase synthesis and deprotection followed the method described in PCT International Publication No. WO2013/027843. That is, after an ammonia aqueous solution and ethanol were added and the mixture was allowed to stand for a while, the solid-phase carrier was filtered and then deprotection of a hydroxyl group was performed using tetrabutylammonium fluoride. The obtained RNA was dissolved using distilled water for injection so as to have a desired concentration.

Thereafter, the obtained crude product of the RNA was purified by column chromatography under the conditions in Table 1. Each of the obtained purified fractions was analyzed by HPLC. Among them, a fraction having a purity of 90% or more was not obtained. As a result of analyzing a fraction having the highest purity, a purity was 87% and a yield was 5%.

Fig. 5 shows the first single-stranded RNA and the second single-stranded RNA, which are used in Example

1, and a linked single-stranded RNA generated by the ligation reaction of the single-stranded RNAs. Fig. 6 shows the first single-stranded RNA and the second single-stranded RNA, which are used in Example 2, and a linked single-stranded RNA generated by the ligation reaction of the single-stranded RNAs. Each abbreviation of "A", "G", "U", and "C" in Sequences 1 to 16 and Figs. 5 and 6 means a unit structure of an RNA containing a ribose ring shown below.

A is represented by the following formula.

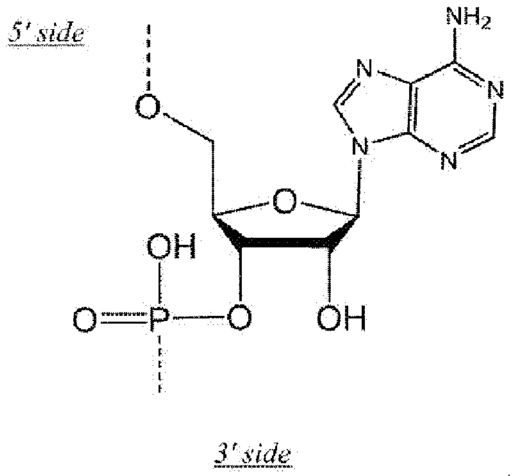


G is represented by the following formula.

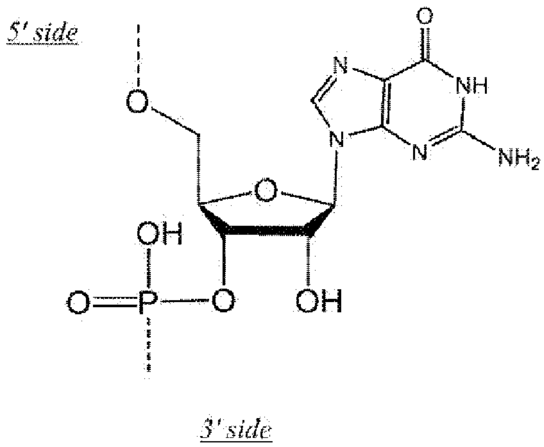


C is represented by the following formula.

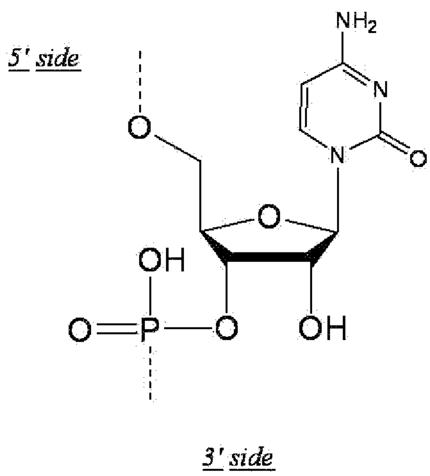

U is represented by the following formula.

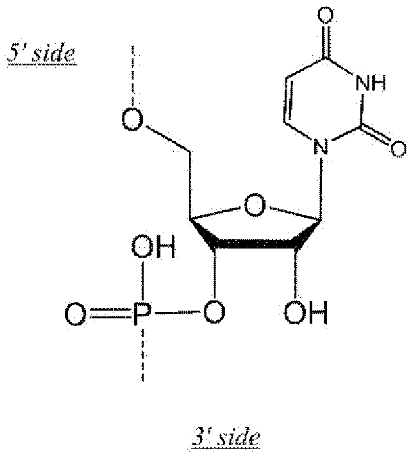


However, in Figs. 5 and 6, only the terminal portion on the 3' side of the RNA strand does not have a phosphate ester portion, and a hydroxyl group is present at a 3-position of ribose.

Each abbreviation of "P" and "K" in Sequences 1 to 16 and Figs. 5 and 6 means a structure of a linker region having an atomic group derived from an amino acid having a structure shown below.

P is represented by the following formula.

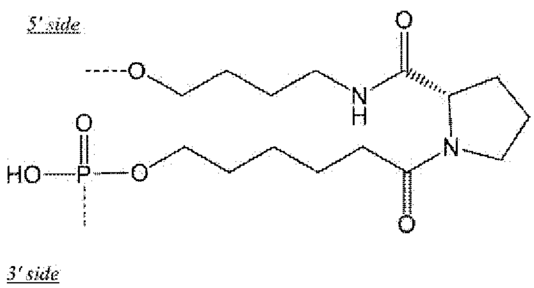


K is represented by the following formula.

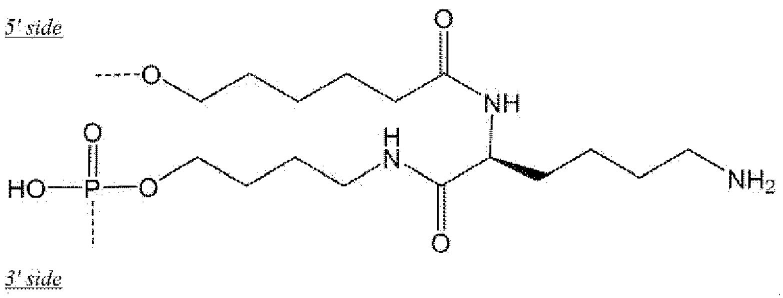


An abbreviation of "p" in Sequences 1 to 16 and Figs. 5 and 6 means that a phosphate group [-O-P(=O)$(OH)_2$] is present at the 5'-position of the ribose ring.

[Industrial Applicability]

The manufacturing method for a single-stranded RNA according to the present invention can be used as a simple manufacturing method for a single-stranded RNA.

## Claims

1. A manufacturing method for a single-stranded RNA, comprising:

   a step of causing an RNA ligase which is classified as EC 6.5.1.3 defined by the International Union of Biochemistry and Molecular Biology as an enzyme number and has a double strand nick repair activity to act on a first single-stranded RNA having a phosphate group at the 5' end and a second single-stranded RNA having a hydroxyl group at the 3' end to link the first single-stranded RNA and the second single-stranded RNA, wherein the manufacturing method comprises the following features a) to g):

   a) the first single-stranded RNA is a single-stranded RNA consisting of an X1 region and a Z region in order from the 5' end;
   b) the second single-stranded RNA is a single strand consisting of an X2 region, a Y2 region, a Ly linker region, and a Y1 region in order from the 5' end;
   c) the X1 region and the X2 region comprise nucleotide sequences which are complementary to each other and have the same number of nucleotides of two or more;
   d) the Y1 region and the Y2 region comprise nucleotide sequences which are complementary to each other and have the same number of nucleotides of two or more;
   e) the Z region is a region which comprises a nucleotide sequence having any number of nucleotides;
   f) the Ly linker region is a linker region having an atomic group derived from an amino acid; and
   g) a single-stranded RNA generated by linking the first single-stranded RNA and the second single-stranded RNA is a linked single-stranded RNA consisting of the X2 region, the Y2 region, the Ly linker region, the Y1 region, the X1 region, and the Z region in order from the 5' end.

2. The manufacturing method according to claim 1,
   wherein the Z region is a region consisting of a Z1 region, an Lz linker region, and a Z2 region in order from the 5' end, the Lz linker region is a linker region having an atomic group derived from an amino acid, and the Z1 region and the Z2 region comprise nucleotide sequences complementary to each other, and a single-stranded RNA generated by linking the first single-stranded RNA and the second single-stranded RNA is a linked single-stranded RNA consisting of the X2 region, the Y2 region, the Ly linker region, the Y1 region, the X1 region, the Z1 region, the Lz linker region, and the Z2 region in order from the 5' end.

3. The manufacturing method according to claim 1, wherein the Ly linker region is a divalent group represented by Formula (I),

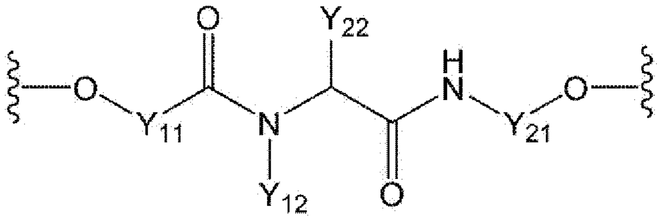

(I)

wherein in the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,
a terminal oxygen atom bonded to $Y_{11}$ is bonded to a phosphorus atom of a phosphate ester of a terminal nucleotide in any one region of the Y1 region and the Y2 region, and
a terminal oxygen atom bonded to $Y_{21}$ is bonded to a phosphorus atom of a phosphate ester of a terminal nucleotide in the other region of the Y1 region and the Y2 region, which is not bonded to $Y_{11}$.

4. The manufacturing method according to claim 2, wherein the Ly linker region is a divalent group represented by Formula (I), and the Lz linker region is a divalent group represented by Formula (I'),

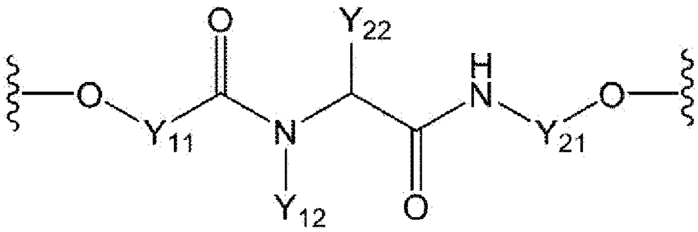

(I)

wherein in the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,
a terminal oxygen atom bonded to $Y_{11}$ is bonded to a phosphorus atom of a phosphate ester of a terminal nucleotide in any one region of the Y1 region and the Y2 region, and
a terminal oxygen atom bonded to $Y_{21}$ is bonded to a phosphorus atom of a phosphate ester of a terminal nucleotide in the other region of the Y2 region and the Y1 region, which is not bonded to $Y_{11}$, and

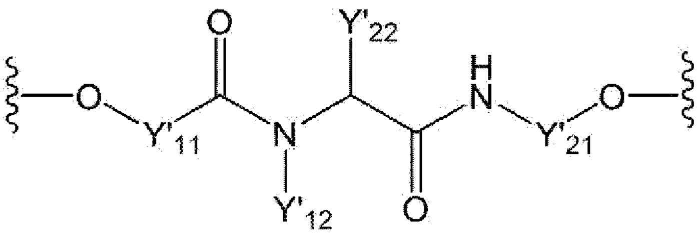

(I')

wherein in the formula, $Y'_{11}$ and $Y'_{21}$ each independently represent an alkylene group having 1 to 20 carbon atoms, and $Y'_{12}$ and $Y'_{22}$ each independently represent a hydrogen atom or an alkyl group which may be substituted with an amino group, or $Y'_{12}$ and $Y'_{22}$ are bonded to each other at their terminals to represent an alkylene group having 3 or 4 carbon atoms,
a terminal oxygen atom bonded to $Y'_{11}$ is bonded to a phosphorus atom of a phosphate ester of a terminal nucleotide in any one region of the Z1 region and the Z2 region, and
a terminal oxygen atom bonded to $Y'_{21}$ is bonded to a phosphorus atom of a phosphate ester of a terminal

nucleotide in the other region of the Z2 region and the Z1 region, which is not bonded to $Y'_{11}$.

**5.** The manufacturing method according to claim 3 or 4, wherein the Ly linker region and the Lz linker region are each independently a divalent group having a structure represented by Formula (II-A) or (II-B),

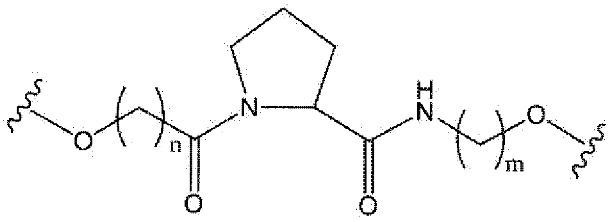

(II-A)

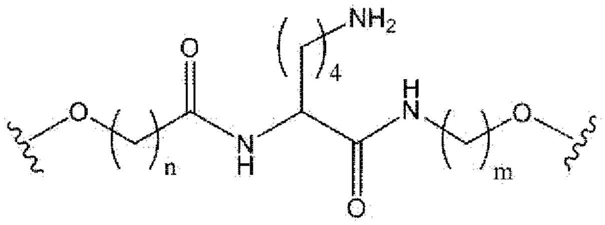

(II-B)

wherein in the formulae, n and m each independently represent any integer of 1 to 20.

**6.** The manufacturing method according to any one of claims 1 to 5, wherein at least one of a W1 region consisting of the X1 region, the Y1 region, and the Z region and a W2 region consisting of the X2 region and the Y2 region includes a nucleotide sequence that suppresses expression of a gene targeted by an RNA interference method.

**7.** The manufacturing method according to any one of claims 1 to 6, wherein the RNA ligase is a T4 bacteriophage-derived T4 RNA ligase 2, a KVP40-derived ligase 2, a Trypanosoma brucei RNA ligase, a Deinococcus radiodurans RNA ligase, or a Leishmania tarentolae RNA ligase.

**8.** The manufacturing method according to any one of claims 1 to 6, wherein the RNA ligase is an RNA ligase consisting of an amino acid sequence having an identity of 95% or more with an amino acid sequence set forth in SEQ ID NO: 21, 22, or 23.

**9.** The manufacturing method according to any one of claims 1 to 6, wherein the RNA ligase is a T4 bacteriophage-derived T4 RNA ligase 2 or a KVP40-derived RNA ligase 2.

FIG. 1

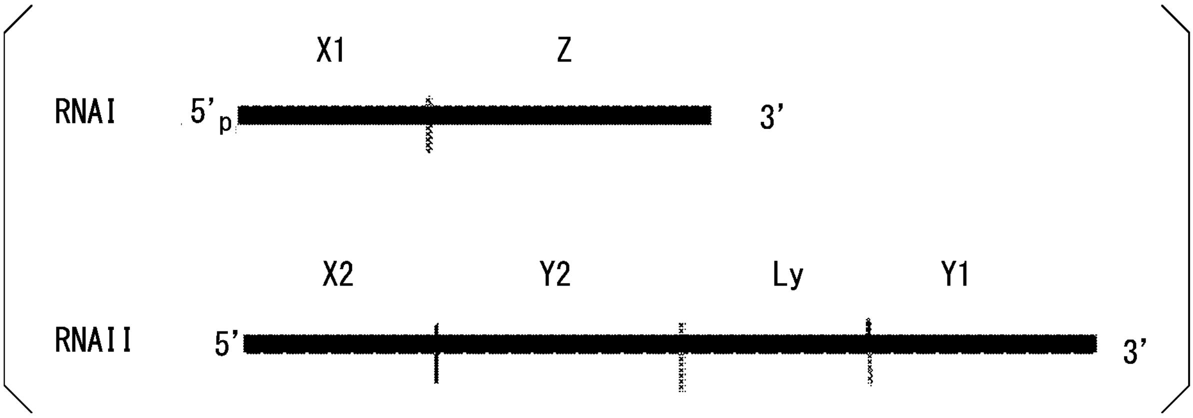
X1
Z
RNAI
5'p
3'
X2
Y2
Ly
Y1
RNAII
5'
3'

FIG. 2

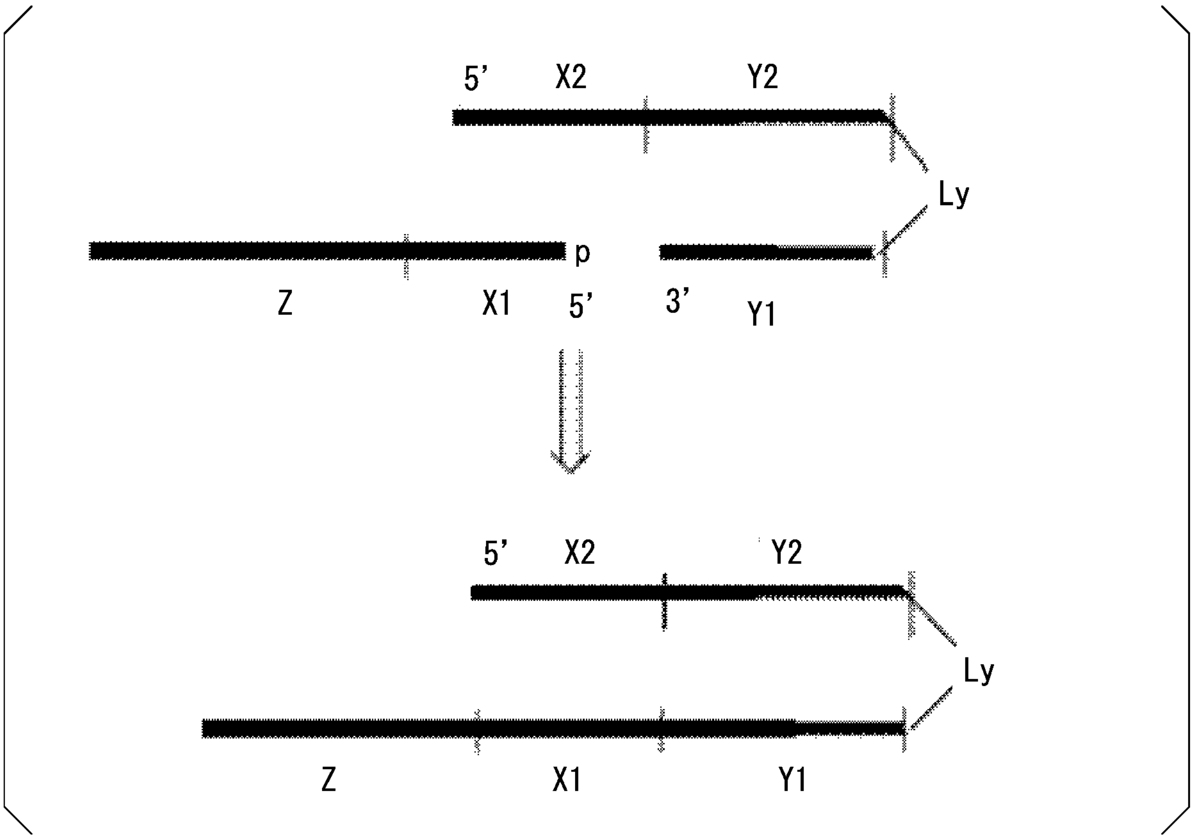
5'
X2
Y2
Ly
p
Z
X1
5'
3'
Y1
5'
X2
Y2
Ly
Z
X1
Y1

FIG. 3

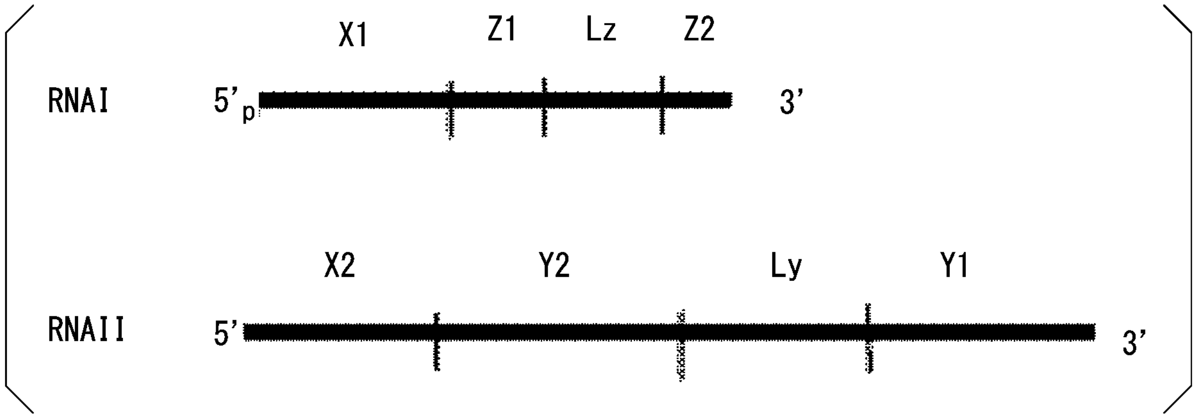
X1
Z1
Lz
Z2
RNAI
5'p
3'
X2
Y2
Ly
Y1
RNAII
5'
3'

FIG. 4

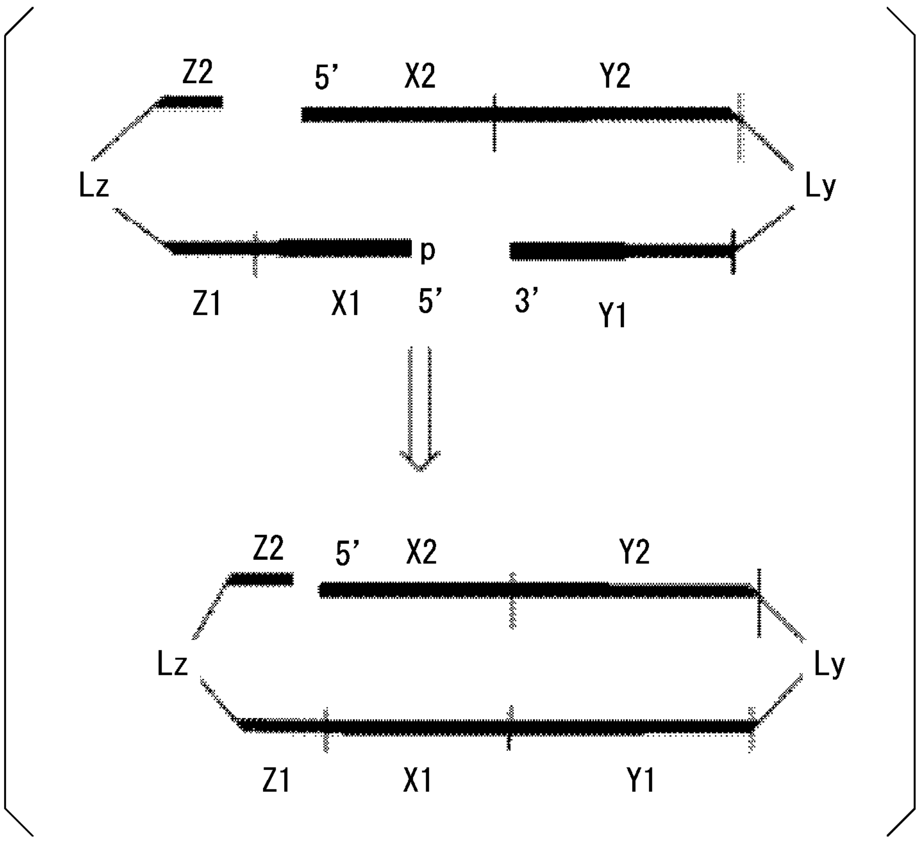
Z2
5'
X2
Y2
Lz
Ly
p
Z1
X1
5'
3'
Y1
Z2
5'
X2
Y2
Lz
Ly
Z1
X1
Y1

FIG. 5

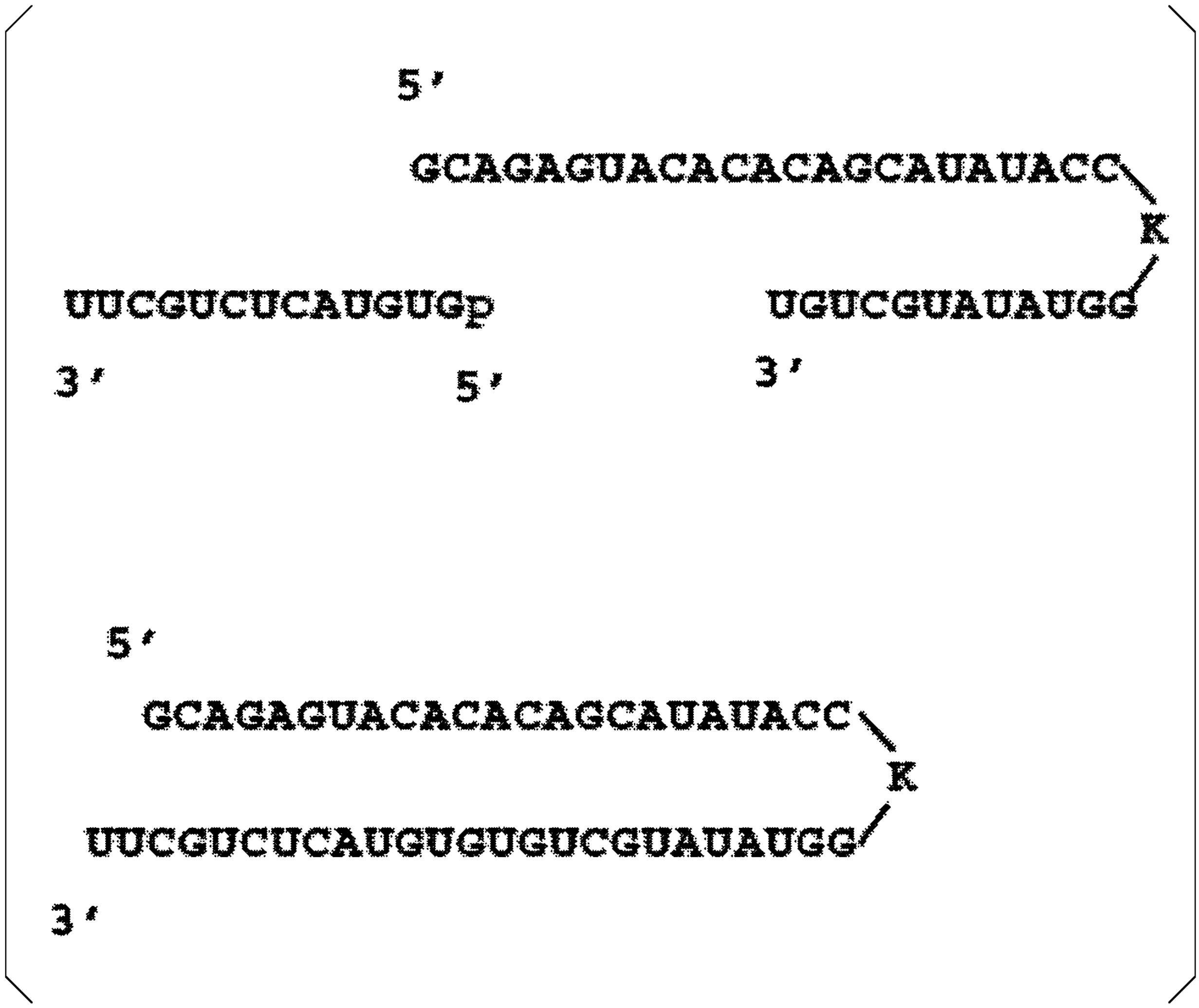
5'
GCAGAGUACACACAGCAUAUACC
K
UUCGUCUCAUGUGp
UGUCGUAUAUGG
3'
5'
3'
5'
GCAGAGUACACACAGCAUAUACC
K
UUCGUCUCAUGUGUGUCGUAUAUGG
3'

FIG. 6

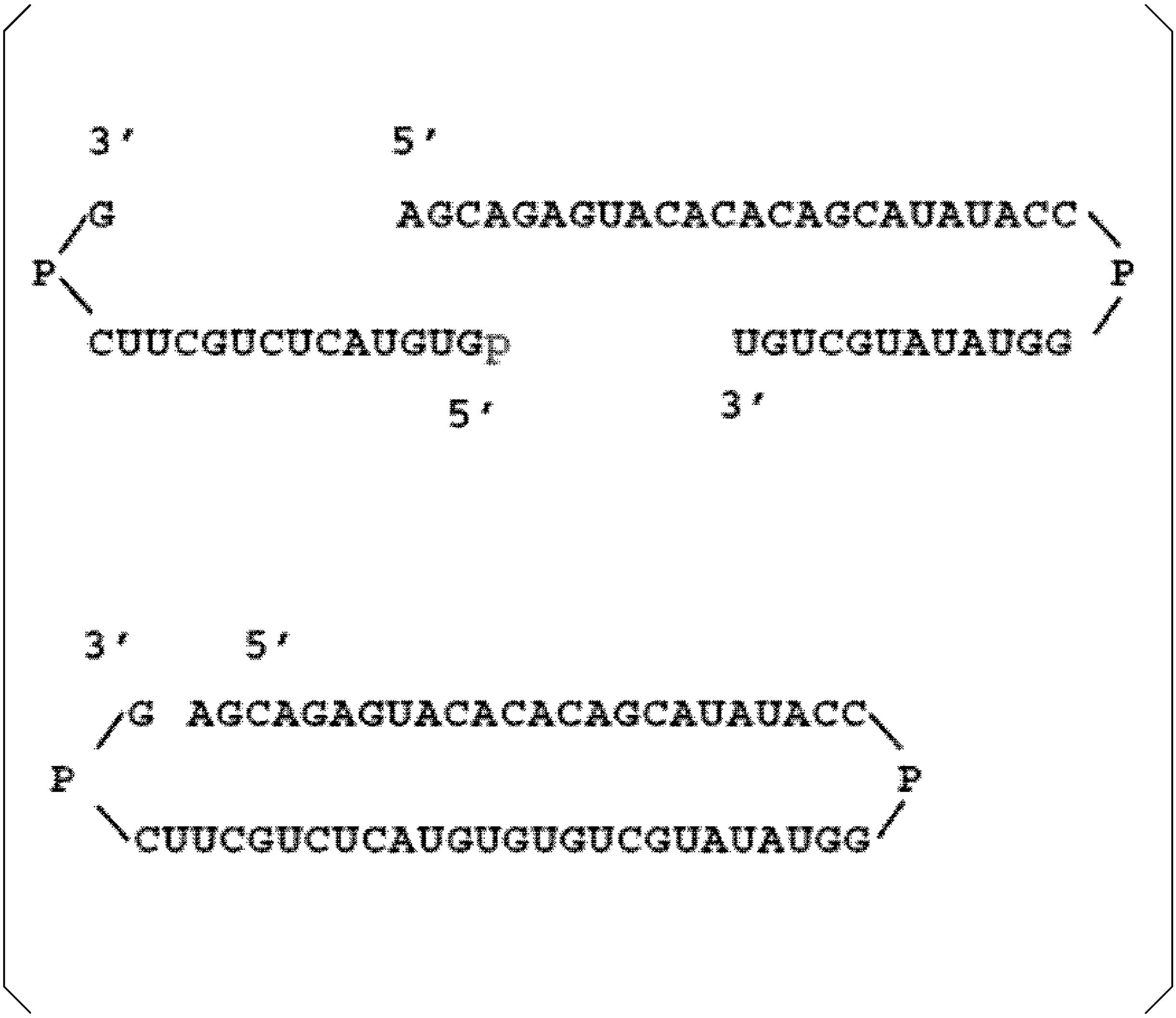
3′
5′
G
AGCAGAGUACACACAGCAUAUACC
P
P
CUUCGUCUCAUGUGp
UGUCGUAUAUGG
5′
3′
3′
5′
G
AGCAGAGUACACACAGCAUAUACC
P
P
CUUCGUCUCAUGUGUGUCGUAUAUGG

## INTERNATIONAL SEARCH REPORT

International application No. PCT/JP2019/003827

A. CLASSIFICATION OF SUBJECT MATTER
Int.Cl. C12P19/34(2006.01)i, C12N15/11(2006.01)i, C12N9/00(2006.01)n, C12N15/52(2006.01)n

According to International Patent Classification (IPC) or to both national classification and IPC

B. FIELDS SEARCHED

Minimum documentation searched (classification system followed by classification symbols)
Int.Cl. C12N15/00-15/90, C12Q1/68-1/6897

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2003-505094 A (PHYLOS, INC.) 12 February 2003, claims, paragraph [0037], fig. 1 & US 2002/0182687 A1, claims, paragraph [0053], fig. 1 & JP 2010-284172 A & US 6429300 B1 & US 2006/0246549 A1 & WO 2001/007657 A1 & EP 1196637 A1 & EP 1870417 A2 & KR 10-2002-0033743 A | 1, 6-9<br>2-5 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 April 2019 (17.04.2019) | 07 May 2019 (07.05.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/003827

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | WO 2008/094599 A2 (ROCKEFELLER UNIVERSITY, THE) 07 August 2008, abstract, SEQ ID NO: 1 & US 2011/0244523 A1 & US 2013/0130322 A1 & US 2015/0010950 A1 | 1, 6-9<br>2-5 |
| Y<br>A | YINS et al., "Characterization of bacteriophage KVP40 and T4 RNA ligase 2", Virology, 2004, 319(1), pp. 141-151, in particular, abstract, SEQ ID NO: 1 | 1, 6-9<br>2-5 |
| Y<br>A | MARTINS, A. et al., "An RNA Ligase from Deinococcus radiodurans", J. Biol. Chem., 2004, 279(49), pp. 50654-50661, in particular, abstract, fig. 1 | 1, 6-9<br>2-5 |
| Y<br>A | WO 2015/099122 A1 (TOKYO MEDICAL UNIVERSITY) 02 July 2015, abstract, paragraph [0035], fig. 1 & US 2017/0037398 A1, in particular, abstract, paragraph [0055], fig. 1 & EP 3088524 A1 & CN 105899663 A | 1, 6-9<br>2-5 |
| A | WO 2013/133221 A1 (BONAC CORPORATION) 12 September 2013, claims & US 2015/0073124 A1, claims & US 2016/0376589 A1 & EP 2824184 A1 & EP 3254700 A1 & CN 104271742 A & CN 108715594 A | 1-9 |
| A | WO 2015/099187 A1 (BONAC CORPORATION) 02 July 2015, claims & US 2016/0319282 A1, claims & JP 2018-145199 A & EP 3088525 A1 & CA 2935022 A & AU 2014370829 A & KR 10-2016-0121510 A & CN 106068324 A & MX 2016008518 A & RU 2016130611 A & BR 112016014986 A & SG 10201805087V A | 1-9 |
| A | WO 2016/104775 A1 (BONAC CORPORATION) 30 June 2016, claims & US 2018/0326091 A1, claims & EP 3239297 A1 & CA 2971827 A & AU 2015368293 A & CN 107109415 A & KR 10-2017-0098914 A & MX 2017008587 A & BR 112017013664 A & SG 11201705223X A & RU 2017126566 A & IL 252880 D | 1-9 |

Form PCT/ISA/210 (continuation of second sheet) (January 2015)

INTERNATIONAL SEARCH REPORT

International application No.
PCT/JP2019/003827

C (Continuation). DOCUMENTS CONSIDERED TO BE RELEVANT

| Category* | Citation of document, with indication, where appropriate, of the relevant passages | Relevant to claim No. |
|---|---|---|
| A | JP 2016-502857 A (NEW ENGLAND BIOLABS, INC.) 01 February 2016, abstract, fig. 1 & US 2014/0179539 A1, abstract, fig. 1 & US 2016/0002716 A1 & WO 2014/100473 A1 & EP 2935624 A1 & EP 3318645 A1 & CN 105121655 A | 1-9 |
| A | WO 2016/010047 A1 (THE UNIVERSITY OF TOKYO) 21 January 2016, abstract, fig. 1 & US 2017/0175177 A1, abstract, fig. 1 & EP 3170898 A1 | 1-9 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018021799 A
- WO 2012017919 PCT
- WO 2013103146 PCT
- WO 2013027843 PCT
- EP 0816368 A
- US 3687808 A


### Non-patent literature cited in the description


- Structure and Mechanism of RNA Ligase. *Structure,* vol. 12, 327-339
- RNA ligase structures reveal the basis for RNA specificity and conformational changes that drive ligation forward. *Cell.,* vol. 127, 71-84
- Characterization of bacteriophage KVP40 and T4 RNA ligase 2. *Virology,* vol. 319, 141-151
- An RNA Ligase from Deinococcus radiodurans. *J Biol Chem.,* vol. 279 (49), 50654-61
- An RNA Ligase from Deinococcus radiodurans. *J Biol Chem.,* vol. 279 (49), 50654-61
- *Assiciation of Two Novel Proteins TbMP52 and TbMP48 with the Trypanosoma brucei RNA Editing Complex,* vol. 21 (2), 380-389
- *The Mitochondrial RNA Ligase from Leishmania tarentolae Can Join RNA Molecules Bridged by a Complementary RNA,* vol. 274 (34), 24289-24296
- *Proc. Natl. Acad. Sci,* 2002, vol. 99 (20), 12709-12714
- Protective Groups in the Chemical Synthesis of Oligoribonucleotides. *Russian Journal of Bioorganic Chemistry,* 2013, vol. 39 (1), 1-21
- Concise Encyclopedia Of Polymer Science And Engineering. John Wiley & Sons, 1990, 858, , 859
- **ENGLISCH et al.** *Angewandte Chemie, International Edition,* 1991, vol. 30, 613